# EUROPÄISCHE PATENTSCHRIFT

(11) **EP 2 609 207 B1**
(45) Veröffentlichungstag und Bekanntmachung des Hinweises auf die Patenterteilung: **30.12.2015**
(21) Anmeldenummer: 11748682.9
(22) Anmeldetag: 26.08.2011
(51) Int. Cl.: C12P 7/24, C12N 9/02, C07K 14/415

(54) **GANZZELL-BIOTRANSFORMATION VON FETTSÄUREN ZU DEN UM EIN KOHLENSTOFFATOM VERKÜRZTEN FETTALDEHYDEN**
WHOLE-CELL BIOTRANSFORMATION OF FATTY ACIDS TO OBTAIN FATTY ALDEHYDES SHORTENED BY ONE CARBON ATOM
BIOTRANSFORMATION DE CELLULE ENTIÈRE D'ACIDES NUCLÉIQUES POUR OBTENIR DES ALDÉHYDES GRAS AYANT UN ATOME DE CARBONE EN MOINS

(30) Priorität: 26.08.2010 US 377237 P; 26.08.2010 DE 102010039833
(43) Veröffentlichungstag der Anmeldung: 03.07.2013
(73) Patentinhaber: Symrise AG, 37603 Holzminden (DE)
(72) Erfinder: SPORLEDER, Fenja, 64560 Riedstadt (DE); BUCHHAUPT, Markus, 61118 Bad Vilbel (DE); SCHRADER, Jens, 60320 Frankfurt am Main (DE)
(74) Vertreter: Eisenführ Speiser
(86) Internationale Anmeldenummer: PCT/EP2011/064762
(87) Internationale Veröffentlichungsnummer: WO 2012/025629

(56) Entgegenhaltungen:
- HAMBERG ET AL: "Alpha-Dioxygenases", BIOCHEMICAL AND BIOPHYSICAL RESEARCH COMMUNICATIONS, Bd. 338, 2005, Seiten 169-174, XP027218277,
- HAMBERG ET AL: "Fatty acid alpha-dioxygenases", PROSTAGLANDINS & OTHER LIPID MEDIATORS, Bd. 68-69, 2002, Seiten 363-374, XP004380634,
- DATABASE PROTEIN [Online] 8. Juni 2010 (2010-06-08), "Os12g0448900 [Oryza sativa Japonica Group]", XP002667560, gefunden im NCBI Database accession no. NP_001066718
- KOEDUKA ET AL: "Catalytic properties of rice alpha-oxygenase", THE JOURNAL OF BIOLOGICAL CHEMISTRY, Bd. 277, 2002, Seiten 22648-22655, XP002667636,
- KAEHNE ET AL: "A recombinant alpha-dioxygenase from rice to produce fatty aldehydes using E. coli", APPLIED MICROBIOLOGY AND BIOTECHNOLOGY, Bd. 90, 24. Februar 2011 (2011-02-24), Seiten 989-995, XP002667333,
- DATABASE GENBANK [Online] AAF64042, 2007 MATSUI ET AL: 'Fatty acid alpha-oxidase (version AAF64042.2)' Database accession no. aaf64042

### SEQUENCE LISTING

<110> Symrise AG
<120> Aldehydherstellung aus Fettsäuren
<130> SA 7322-03WO
<160> 4
<170> PatentIn version 3.3
<210> 1
   <211> 231
   <212> PRT
   <213> Oryza sativa
<400> 1
   Gly Pro Ile Leu Gly Gly Leu Val Gly Leu Lys Lys Pro Asn Asn His
   1               5                   10                  15
   Gly Val Pro Tyr Ser Leu Thr Glu Glu Phe Thr Ser Val Tyr Arg Met
               20                  25                  30
   His Ser Leu Ile Pro Ser Thr Leu Lys Leu Arg Asp Pro Thr Gly Gln
           35                  40                  45
   Pro Asp Ala Asn Asn Ser Pro Pro Cys Leu Glu Asp Ile Asp Ile Gly
       50                  55                  60
   Glu Met Ile Gly Leu Lys Gly Glu Glu Gln Leu Ser Lys Ile Gly Phe
   65                  70                  75                  80
   Glu Lys Gln Ala Leu Ser Met Gly Tyr Gln Ala Cys Gly Ala Leu Glu
                   85                  90                  95
   Leu Trp Asn Tyr Pro Ser Phe Phe Arg Asn Leu Ile Pro Gln Asn Leu
               100                 105                 110
   Asp Gly Thr Asn Arg Ser Asp Arg Ile Asp Leu Ala Ala Leu Glu Val
           115                 120                 125
   Tyr Arg Asp Arg Glu Arg Ser Val Pro Arg Tyr Asn Glu Phe Arg Arg
       130                 135                 140
   Arg Leu Phe Leu Ile Pro Ile Lys Ser Trp Glu Asp Leu Thr Ser Asp
   145                 150                 155                 160
   Lys Asp Ala Ile Glu Thr Ile Arg Ala Ile Tyr Gly Asp Asp Val Glu
                   165                 170                 175
   Lys Leu Asp Leu Leu Val Gly Leu Met Ala Glu Lys Lys Ile Lys Gly
               180                 185                 190
   Phe Ala Ile Ser Glu Thr Ala Phe Asn Ile Phe Ile Leu Met Ala Ser
           195                 200                 205
   Arg Arg Leu Glu Ala Asp Arg Phe Phe Thr Ser Asn Phe Asn Glu Glu
       210                 215                 220
   Thr Tyr Thr Lys Lys Gly Met
   225                 230
<210> 2
   <211> 618
   <212> PRT
   <213> Oryza sativa
<400> 2
   Met Gly Ser Gly Leu Phe Lys Pro Arg Val His Pro Asp Leu Arg Asp
   1               5                   10                  15
   Val Phe Ser Lys Met Ser Phe Phe Asp Lys Ile Gly Phe Leu Phe Ile
               20                  25                  30
   His Ala Phe Asp Lys Arg Asn Leu Trp His Lys Val Pro Val Pro Ile
           35                  40                  45
   Gly Leu Leu Tyr Leu Asn Thr Arg Arg Thr Leu Leu Glu Lys Tyr Asn
       50                  55                  60
   Leu Leu Ala Val Gly Arg Ser Ser His Gly Ala Leu Phe Asp Pro Lys
   65                  70                  75                  80
   Glu Phe Leu Tyr Arg Thr Glu Asp Gly Lys Tyr Asn Asp Pro His Asn
                   85                  90                  95
   Ala Glu Ala Gly Ser Gln Asn Thr Phe Phe Gly Arg Asn Met Glu Pro
               100                 105                 110
   Val Asp Gln Gln Asp Glu Leu Met Ser Pro Asp Pro Phe Val Val Ala
           115                 120                 125
   Thr Lys Leu Leu Ala Arg Arg Glu Tyr Lys Asp Thr Gly Lys Gln Phe
       130                 135                 140
   Asn Ile Leu Ala Ala Ala Trp Ile Gln Phe Met Val His Asp Trp Met
   145                 150                 155                 160
   Asp His Met Glu Asp Thr Gly Gln Ile Gly Ile Thr Ala Pro Lys Glu
   165 170 175
   Val Ala Asn Glu Cys Pro Leu Lys Ser Phe Lys Phe His Pro Thr Lys
   180 185 190
   Glu Leu Pro Thr Asn Ser Asp Gly Ile Lys Ile Gly His Tyr Asn Ile
   195 200 205
   Arg Thr Ala Trp Trp Asp Gly Ser Ala Val Tyr Gly Asn Asn Glu Glu
   210 215 220
   Arg Ala Glu Lys Leu Arg Thr Tyr Val Asp Gly Lys Leu Val Ile Gly
   225 230 235 240
   Asp Asp Gly Leu Leu Leu His Lys Glu Asn Gly Val Ala Leu Ser Gly
   245 250 255
   Asp Ile Arg Asn Ser Trp Ala Gly Val Ser Ile Leu Gln Ala Leu Phe
   260 265 270
   Val Lys Glu His Asn Ala Val Cys Asp Ala Ile Lys Glu Glu His Pro
   275 280 285
   Asn Leu Ser Asp Glu Glu Leu Tyr Arg Tyr Ala Lys Leu Val Thr Ser
   290 295 300
   Ala Val Ile Ala Lys Val His Thr Ile Asp Trp Thr Val Glu Leu Leu
   305 310 315 320
   Lys Thr Lys Thr Met Arg Ala Ala Met Arg Ala Asn Trp Tyr Gly Leu
   325 330 335
   Leu Gly Lys Lys Ile Lys Asp Thr Phe Gly His Ile Gly Gly Pro Ile
   340 345 350
   Leu Gly Gly Leu Val Gly Leu Lys Lys Pro Asn Asn His Gly Val Pro
   355 360 365
   Tyr Ser Leu Thr Glu Glu Phe Thr Ser Val Tyr Arg Met His Ser Leu
   370 375 380
   Ile Pro Ser Thr Leu Lys Leu Arg Asp Pro Thr Gly Gln Pro Asp Ala
   385 390 395 400
   Asn Asn Ser Pro Pro Cys Leu Glu Asp Ile Asp Ile Gly Glu Met Ile
   405 410 415
   Gly Leu Lys Gly Glu Glu Gln Leu Ser Lys Ile Gly Phe Glu Lys Gln
   420 425 430
   Ala Leu Ser Met Gly Tyr Gln Ala Cys Gly Ala Leu Glu Leu Trp Asn
   435 440 445
   Tyr Pro Ser Phe Phe Arg Asn Leu Ile Pro Gln Asn Leu Asp Gly Thr
   450 455 460
   Asn Arg Ser Asp Arg Ile Asp Leu Ala Ala Leu Glu Val Tyr Arg Asp
   465 470 475 480
   Arg Glu Arg Ser Val Pro Arg Tyr Asn Glu Phe Arg Arg Arg Leu Phe
   485 490 495
   Leu Ile Pro Ile Lys Ser Trp Glu Asp Leu Thr Ser Asp Lys Asp Ala
   500 505 510
   Ile Glu Thr Ile Arg Ala Ile Tyr Gly Asp Asp Val Glu Lys Leu Asp
   515 520 525
   Leu Leu Val Gly Leu Met Ala Glu Lys Lys Ile Lys Gly Phe Ala Ile
   530 535 540
   Ser Glu Thr Ala Phe Asn Ile Phe Ile Leu Met Ala Ser Arg Arg Leu
   545 550 555 560
   Glu Ala Asp Arg Phe Phe Thr Ser Asn Phe Asn Glu Glu Thr Tyr Thr
   565 570 575
   Lys Lys Gly Met Gln Trp Val Lys Thr Thr Glu Gly Leu Arg Asp Val
   580 585 590
   Ile Asn Arg His Tyr Pro Glu Ile Thr Ala Lys Trp Met Lys Ser Ser
   595 600 605
   Ser Ala Phe Ser Val Trp Asp Ala Asp Tyr
   610 615
<210> 3
   <211> 1854
   <212> DNA
   <213> Oryza sativa
<220>
   <221> misc_feature
   <222> (6)..(6)
   <223> n is a, c, g, or t
<220>
   <221> misc_feature
   <222> (9)..(9)
   <223> n is a, c, g, or t
<220>
   <221> misc_feature
   <222> (12)..(12)
   <223> n is a, c, g, or t
<220>
   <221> misc_feature
   <222> (15)..(15)
   <223> n is a, c, g, or t
<220>
   <221> misc_feature
   <222> (24)..(24)
   <223> n is a, c, g, or t
<220>
   <221> misc_feature
   <222> (27)..(27)
   <223> n is a, c, g, or t
<220>
   <221> misc_feature
   <222> (30)..(30)
   <223> n is a, c, g, or t
<220>
   <221> misc_feature
   <222> (36)..(36)
   <223> n is a, c, g, or t
<220>
   <221> misc_feature
   <222> (42)..(42)
   <223> n is a, c, g, or t
<220>
   <221> misc_feature
   <222> (45)..(45)
   <223> n is a, c, g, or t
<220>
   <221> misc_feature
   <222> (51)..(51)
   <223> n is a, c, g, or t
<220>
   <221> misc_feature
   <222> (57)..(57)
   <223> n is a, c, g, or t
<220>
   <221> misc_feature
   <222> (66)..(66)
   <223> n is a, c, g, or t
<220>
   <221> misc_feature
   <222> (84)..(84)
   <223> n is a, c, g, or t
<220>
   <221> misc_feature
   <222> (90)..(90)
   <223> n is a, c, g, or t
<220>
   <221> misc_feature
   <222> (102)..(102)
   <223> n is a, c, g, or t
<220>
   <221> misc_feature
   <222> (114)..(114)
   <223> n is a, c, g, or t
<220>
   <221> misc_feature
   <222> (120)..(120)
   <223> n is a, c, g, or t
<220>
   <221> misc_feature
   <222> (132)..(132)
   <223> n is a, c, g, or t
<220>
   <221> misc_feature
   <222> (135)..(135)
   <223> n is a, c, g, or t
<220>
   <221> misc_feature
   <222> (138)..(138)
   <223> n is a, c, g, or t
<220>
   <221> misc_feature
   <222> (141)..(141)
   <223> n is a, c, g, or t
<220>
   <221> misc_feature
   <222> (147)..(147)
   <223> n is a, c, g, or t
<220>
   <221> misc_feature
   <222> (150)..(150)
   <223> n is a, c, g, or t
<220>
   <221> misc_feature
   <222> (153)..(153)
   <223> n is a, c, g, or t
<220>
   <221> misc_feature
   <222> (159)..(159)
   <223> n is a, c, g, or t
<220>
   <221> misc_feature
   <222> (165)..(165)
   <223> n is a, c, g, or t
<220>
   <221> misc_feature
   <222> (168)..(168)
   <223> n is a, c, g, or t
<220>
   <221> misc_feature
   <222> (171)..(171)
   <223> n is a, c, g, or t
<220>
   <221> misc_feature
   <222> (174)..(174)
   <223> n is a, c, g, or t
<220>
   <221> misc_feature
   <222> (177)..(177)
   <223> n is a, c, g, or t
<220>
   <221> misc_feature
   <222> (180)..(180)
   <223> n is a, c, g, or t
<220>
   <221> misc_feature
   <222> (195)..(195)
   <223> n is a, c, g, or t
<220>
   <221> misc_feature
   <222> (198)..(198)
   <223> n is a, c, g, or t
<220>
   <221> misc_feature
   <222> (201)..(201)
   <223> n is a, c, g, or t
<220>
   <221> misc_feature
   <222> (204)..(204)
   <223> n is a, c, g, or t
<220>
   <221> misc_feature
   <222> (207)..(207)
   <223> n is a, c, g, or t
<220>
   <221> misc_feature
   <222> (210)..(210)
   <223> n is a, c, g, or t
<220>
   <221> misc_feature
   <222> (213)..(213)
   <223> n is a, c, g, or t
<220>
   <221> misc_feature
   <222> (216)..(216)
   <223> n is a, c, g, or t
<220>
   <221> misc_feature
   <222> (222)..(222)
   <223> n is a, c, g, or t
<220>
   <221> misc_feature
   <222> (225)..(225)
   <223> n is a, c, g, or t
<220>
   <221> misc_feature
   <222> (228)..(228)
   <223> n is a, c, g, or t
<220>
   <221> misc_feature
   <222> (237)..(237)
   <223> n is a, c, g, or t
<220>
   <221> misc_feature
   <222> (249)..(249)
   <223> n is a, c, g, or t
<220>
   <221> misc_feature
   <222> (255) .. (255)
   <223> n is a, c, g, or t
<220>
   <221> misc_feature
   <222> (258) .. (258)
   <223> n is a, c, g, or t
<220>
   <221> misc_feature
   <222> (267)..(267)
   <223> n is a, c, g, or t
<220>
   <221> misc_feature
   <222> (282)..(282)
   <223> n is a, c, g, or t
<220>
   <221> misc_feature
   <222> (291)..(291)
   <223> n is a, c, g, or t
<220>
   <221> misc_feature
   <222> (297)..(297)
   <223> n is a, c, g, or t
<220>
   <221> misc_feature
   <222> (300)..(300)
   <223> n is a, c, g, or t
<220>
   <221> misc_feature
   <222> (303)..(303)
   <223> n is a, c, g, or t
<220>
   <221> misc_feature
   <222> (312)..(312)
   <223> n is a, c, g, or t
<220>
   <221> misc_feature
   <222> (321)..(321)
   <223> n is a, c, g, or t
<220>
   <221> misc_feature
   <222> (324)..(324)
   <223> n is a, c, g, or t
<220>
   <221> misc_feature
   <222> (336)..(336)
   <223> n is a, c, g, or t
<220>
   <221> misc_feature
   <222> (339)..(339)
   <223> n is a, c, g, or t
<220>
   <221> misc_feature
   <222> (357)..(357)
   <223> n is a, c, g, or t
<220>
   <221> misc_feature
   <222> (363)..(363)
   <223> n is a, c, g, or t
<220>
   <221> misc_feature
   <222> (366)..(366)
   <223> n is a, c, g, or t
<220>
   <221> misc_feature
   <222> (372)..(372)
   <223> n is a, c, g, or t
<220>
   <221> misc_feature
   <222> (378)..(378)
   <223> n is a, c, g, or t
<220>
   <221> misc_feature
   <222> (381)..(381)
   <223> n is a, c, g, or t
<220>
   <221> misc_feature
   <222> (384)..(384)
   <223> n is a, c, g, or t
<220>
   <221> misc_feature
   <222> (387)..(387)
   <223> n is a, c, g, or t
<220>
   <221> misc_feature
   <222> (393)..(393)
   <223> n is a, c, g, or t
<220>
   <221> misc_feature
   <222> (396)..(396)
   <223> n is a, c, g, or t
<220>
   <221> misc_feature
   <222> (399)..(399)
   <223> n is a, c, g, or t
<220>
   <221> misc_feature
   <222> (402)..(402)
   <223> n is a, c, g, or t
<220>
   <221> misc_feature
   <222> (405)..(405)
   <223> n is a, c, g, or t
<220>
   <221> misc_feature
   <222> (420)..(420)
   <223> n is a, c, g, or t
<220>
   <221> misc_feature
   <222> (423)..(423)
   <223> n is a, c, g, or t
<220>
   <221> misc_feature
   <222> (441)..(441)
   <223> n is a, c, g, or t
<220>
   <221> misc_feature
   <222> (444)..(444)
   <223> n is a, c, g, or t
<220>
   <221> misc_feature
   <222> (447)..(447)
   <223> n is a, c, g, or t
<220>
   <221> misc_feature
   <222> (450)..(450)
   <223> n is a, c, g, or t
<220>
   <221> misc_feature
   <222> (468)..(468)
   <223> n is a, c, g, or t
<220>
   <221> misc_feature
   <222> (498)..(498)
   <223> n is a, c, g, or t
<220>
   <221> misc_feature
   <222> (501)..(501)
   <223> n is a, c, g, or t
<220>
   <221> misc_feature
   <222> (510)..(510)
   <223> n is a, c, g, or t
<220>
   <221> misc_feature
   <222> (516)..(516)
   <223> n is a, c, g, or t
<220>
   <221> misc_feature
   <222> (519)..(519)
   <223> n is a, c, g, or t
<220>
   <221> misc_feature
   <222> (522)..(522)
   <223> n is a, c, g, or t
<220>
   <221> misc_feature
   <222> (531)..(531)
   <223> n is a, c, g, or t
<220>
   <221> misc_feature
   <222> (534)..(534)
   <223> n is a, c, g, or t
<220>
   <221> misc_feature
   <222> (546)..(546)
   <223> n is a, c, g, or t
<220>
   <221> misc_feature
   <222> (549)..(549)
   <223> n is a, c, g, or t
<220>
   <221> misc_feature
   <222> (555)..(555)
   <223> n is ä, c, g, or t
<220>
   <221> misc_feature
   <222> (570)..(570)
   <223> n is a, c, g, or t
<220>
   <221> misc_feature
   <222> (573)..(573)
   <223> n is a, c, g, or t
<220>
   <221> misc_feature
   <222> (582)..(582)
   <223> n is a, c, g, or t
<220>
   <221> misc_feature
   <222> (585)..(585)
   <223> n is a, c, g, or t
<220>
   <221> misc_feature
   <222> (588) .. (588)
   <223> n is a, c, g, or t
<220>
   <221> misc_feature
   <222> (594)..(594)
   <223> n is a, c, g, or t
<220>
   <221> misc_feature
   <222> (600)..(600)
   <223> n is a, c, g, or t
<220>
   <221> misc_feature
   <222> (612)..(612)
   <223> n is a, c, g, or t
<220>
   <221> misc_feature
   <222> (627)..(627)
   <223> n is a, c, g, or t
<220>
   <221> misc_feature
   <222> (630)..(630)
   <223> n is a, c, g, or t
<220>
   <221> misc_feature
   <222> (633)..(633)
   <223> n is a, c, g, or t
<220>
   <221> misc_feature
   <222> (645)..(645)
   <223> n is a, c, g, or t
<220>
   <221> misc_feature
   <222> (648)..(648)
   <223> n is a, c, g, or t
<220>
   <221> misc_feature
   <222> (651)..(651)
   <223> n is a, c, g, or t
<220>
   <221> misc_feature
   <222> (654)..(654)
   <223> n is a, c, g, or t
<220>
   <221> misc_feature
   <222> (660)..(660)
   <223> n is a, c, g, or t
<220>
   <221> misc_feature
   <222> (675)..(675)
   <223> n is a, c, g, or t
<220>
   <221> misc_feature
   <222> (678)..(678)
   <223> n is a, c, g, or t
<220>
   <221> misc_feature
   <222> (687)..(687)
   <223> n is a, c, g, or t
<220>
   <221> misc_feature
   <222> (690)..(690)
   <223> n is a, c, g, or t
<220>
   <221> misc_feature
   <222> (693)..(693)
   <223> n is a, c, g, or t
<220>
   <221> misc_feature
   <222> (699)..(699)
   <223> n is a, c, g, or t
<220>
   <221> misc_feature
   <222> (705)..(705)
   <223> n is a, c, g, or t
<220>
   <221> misc_feature
   <222> (711)..(711)
   <223> n is a, c, g, or t
<220>
   <221> misc_feature
   <222> (714)..(714)
   <223> n is a, c, g, or t
<220>
   <221> misc_feature
   <222> (720)..(720)
   <223> n is a, c, g, or t
<220>
   <221> misc_feature
   <222> (729)..(729)
   <223> n is a, c, g, or t
<220>
   <221> misc_feature
   <222> (732)..(732)
   <223> n is a, c, g, or t
<220>
   <221> misc_feature
   <222> (735)..(735)
   <223> n is a, c, g, or t
<220>
   <221> misc_feature
   <222> (738)..(738)
   <223> n is a, c, g, or t
<220>
   <221> misc_feature
   <222> (753)..(753)
   <223> n is a, c, g, or t
<220>
   <221> misc_feature
   <222> (756)..(756)
   <223> n is a, c, g, or t
<220>
   <221> misc_feature
   <222> (759)..(759)
   <223> n is a, c, g, or t
<220>
   <221> misc_feature
   <222> (762)..(762)
   <223> n is a, c, g, or t
<220>
   <221> misc_feature
   <222> (765)..(765)
   <223> n is a, c, g, or t
<220>
   <221> misc_feature
   <222> (768)..(768)
   <223> n is a, c, g, or t
<220>
   <221> misc_feature
   <222> (777)..(777)
   <223> n is a, c, g, or t
<220>
   <221> misc_feature
   <222> (783)..(783)
   <223> n is a, c, g, or t
<220>
   <221> misc_feature
   <222> (789)..(789)
   <223> n is a, c, g, or t
<220>
   <221> misc_feature
   <222> (792)..(792)
   <223> n is a, c, g, or t
<220>
   <221> misc_feature
   <222> (795)..(795)
   <223> n is a, c, g, or t
<220>
   <221> misc_feature
   <222> (798)..(798)
   <223> n is a, c, g, or t
<220>
   <221> misc_feature
   <222> (804)..(804)
   <223> n is a, c, g, or t
<220>
   <221> misc_feature
   <222> (810)..(810)
   <223> n is a, c, g, or t
<220>
   <221> misc_feature
   <222> (813)..(813)
   <223> n is a, c, g, or t
<220>
   <221> misc_feature
   <222> (819)..(819)
   <223> n is a, c, g, or t
<220>
   <221> misc_feature
   <222> (834)..(834)
   <223> n is a, c, g, or t
<220>
   <221> misc_feature
   <222> (837)..(837)
   <223> n is a, c, g, or t
<220>
   <221> misc_feature
   <222> (846)..(846)
   <223> n is a, c, g, or t
<220>
   <221> misc_feature
   <222> (864)..(864)
   <223> n is a, c, g, or t
<220>
   <221> misc_feature
   <222> (870)..(870)
   <223> n is a, c, g, or t
<220>
   <221> misc_feature
   <222> (873)..(873)
   <223> n is a, c, g, or t
<220>
   <221> misc_feature
   <222> (885)..(885)
   <223> n is a, c, g, or t
<220>
   <221> misc_feature
   <222> (891)..(891)
   <223> n is a, c, g, or t
<220>
   <221> misc_feature
   <222> (897)..(897)
   <223> n is a, c, g, or t
<220>
   <221> misc_feature
   <222> (903)..(903)
   <223> n is a, c, g, or t
<220>
   <221> misc_feature
   <222> (906)..(906)
   <223> n is a, c, g, or t
<220>
   <221> misc_feature
   <222> (909)..(909)
   <223> n is a, c, g, or t
<220>
   <221> misc_feature
   <222> (912)..(912)
   <223> n is a, c, g, or t
<220>
   <221> misc_feature
   <222> (915)..(915)
   <223> n is a, c, g, or t
<220>
   <221> misc_feature
   <222> (918)..(918)
   <223> n is a, c, g, or t
<220>
   <221> misc_feature
   <222> (924)..(924)
   <223> n is a, c, g, or t
<220>
   <221> misc_feature
   <222> (930)..(930)
   <223> n is a, c, g, or t
<220>
   <221> misc_feature
   <222> (936)..(936)
   <223> n is a, c, g, or t
<220>
   <221> misc_feature
   <222> (948)..(948)
   <223> n is a, c, g, or t
<220>
   <221> misc_feature
   <222> (951)..(951)
   <223> n is a, c, g, or t
<220>
   <221> misc_feature
   <222> (957)..(957)
   <223> n is a, c, g, or t
<220>
   <221> misc_feature
   <222> (960)..(960)
   <223> n is a, c, g, or t
<220>
   <221> misc_feature
   <222> (966)..(966)
   <223> n is a, c, g, or t
<220>
   <221> misc_feature
   <222> (972)..(972)
   <223> n is a, c, g, or t
<220>
   <221> misc_feature
   <222> (978)..(978)
   <223> n is a, c, g, or t
<220>
   <221> misc_feature
   <222> (981)..(981)
   <223> n is a, c, g, or t
<220>
   <221> misc_feature
   <222> (984)..(984)
   <223> n is a, c, g, or t
<220>
   <221> misc_feature
   <222> (990)..(990)
   <223> n is a, c, g, or t
<220>
   <221> misc_feature
   <222> (993)..(993)
   <223> n is a, c, g, or t
<220>
   <221> misc_feature
   <222> (1005)..(1005)
   <223> n is a, c, g, or t
<220>
   <221> misc_feature
   <222> (1008)..(1008)
   <223> n is a, c, g, or t
<220>
   <221> misc_feature
   <222> (1011)..(1011)
   <223> n is a, c, g, or t
<220>
   <221> misc_feature
   <222> (1014)..(1014)
   <223> n is a, c, g, or t
<220>
   <221> misc_feature
   <222> (1032)..(1032)
   <223> n is a, c, g, or t
<220>
   <221> misc_feature
   <222> (1038)..(1038)
   <223> n is a, c, g, or t
<220>
   <221> misc_feature
   <222> (1047)..(1047)
   <223> n is a, c, g, or t
<220>
   <221> misc_feature
   <222> (1050)..(1050)
   <223> n is a, c, g, or t
<220>
   <221> misc_feature
   <222> (1053)..(1053)
   <223> n is a, c, g, or t
<220>
   <221> misc_feature
   <222> (1059)..(1059)
   <223> n is a, c, g, or t
<220>
   <221> misc_feature
   <222> (1062)..(1062)
   <223> n is a, c, g, or t
<220>
   <221> misc_feature
   <222> (1065)..(1065)
   <223> n is a, c, g, or t
<220>
   <221> misc_feature
   <222> (1068)..(1068)
   <223> n is a, c, g, or t
<220>
   <221> misc_feature
   <222> (1071)..(1071)
   <223> n is a, c, g, or t
<220>
   <221> misc_feature
   <222> (1074)..(1074)
   <223> n is a, c, g, or t
<220>
   <221> misc_feature
   <222> (1077)..(1077)
   <223> n is a, c, g, or t
<220>
   <221> misc_feature
   <222> (1086)..(1086)
   <223> n is a, c, g, or t
<220>
   <221> misc_feature
   <222> (1098)..(1098)
   <223> n is a, c, g, or t
<220>
   <221> misc_feature
   <222> (1101)..(1101)
   <223> n is a, c, g, or t
<220>
   <221> misc_feature
   <222> (1104)..(1104)
   <223> n is a, c, g, or t
<220>
   <221> misc_feature
   <222> (1110)..(1110)
   <223> n is a, c, g, or t
<220>
   <221> misc_feature
   <222> (1113)..(1113)
   <223> n is a, c, g, or t
<220>
   <221> misc_feature
   <222> (1116)..(1116)
   <223> n is a, c, g, or t
<220>
   <221> misc_feature
   <222> (1128)..(1128)
   <223> n is a, c, g, or t
<220>
   <221> misc_feature
   <222> (1131)..(1131)
   <223> n is a, c, g, or t
<220>
   <221> misc_feature
   <222> (1134)..(1134)
   <223> n is a, c, g, or t
<220>
   <221> misc_feature
   <222> (1140)..(1140)
   <223> n is a, c, g, or t
<220>
   <221> misc_feature
   <222> (1149)..(1149)
   <223> n is a, c, g, or t
<220>
   <221> misc_feature
   <222> (1152)..(1152)
   <223> n is a, c, g, or t
<220>
   <221> misc_feature
   <222> (1158)..(1158)
   <223> n is a, c, g, or t
<220>
   <221> misc_feature
   <222> (1161)..(1161)
   <223> n is a, c, g, or t
<220>
   <221> misc_feature
   <222> (1164)..(1164)
   <223> n is a, c, g, or t
<220>
   <221> misc_feature
   <222> (1167)..(1167)
   <223> n is a, c, g, or t
<220>
   <221> misc_feature
   <222> (1173)..(1173)
   <223> n is a, c, g, or t
<220>
   <221> misc_feature
   <222> (1176)..(1176)
   <223> n is a, c, g, or t
<220>
   <221> misc_feature
   <222> (1182)..(1182)
   <223> n is a, c, g, or t
<220>
   <221> misc_feature
   <222> (1185)..(1185)
   <223> n is a, c, g, or t
<220>
   <221> misc_feature
   <222> (1188)..(1188)
   <223> n is a, c, g, or t
<220>
   <221> misc_feature
   <222> (1194)..(1194)
   <223> n is a, c, g, or t
<220>
   <221> misc_feature
   <222> (1200)..(1200)
   <223> n is a, c, g, or t
<220>
   <221> misc_feature
   <222> (1209)..(1209)
   <223> n is a, c, g, or t
<220>
   <221> misc_feature
   <222> (1212)..(1212)
   <223> n is a, c, g, or t
<220>
   <221> misc_feature
   <222> (1215)..(1215)
   <223> n is a, c, g, or t
<220>
   <221> misc_feature
   <222> (1221)..(1221)
   <223> n is a, c, g, or t
<220>
   <221> misc_feature
   <222> (1239)..(1239)
   <223> n is a, c, g, or t
<220>
   <221> misc_feature
   <222> (1251) .. (1251)
   <223> n is a, c, g, or t
<220>
   <221> misc_feature
   <222> (1254)..(1254)
   <223> n is a, c, g, or t
<220>
   <221> misc_feature
   <222> (1260)..(1260)
   <223> n is a, c, g, or t
<220>
   <221> misc_feature
   <222> (1272)..(1272)
   <223> n is a, c, g, or t
<220>
   <221> misc_feature
   <222> (1275)..(1275)
   <223> n is a, c, g, or t
<220>
   <221> misc_feature
   <222> (1284)..(1284)
   <223> n is a, c, g, or t
<220>
   <221> misc_feature
   <222> (1299)..(1299)
   <223> n is a, c, g, or t
<220>
   <221> misc_feature
   <222> (1302)..(1302)
   <223> n is a, c, g, or t
<220>
   <221> misc_feature
   <222> (1305)..(1305)
   <223> n is a, c, g, or t
<220>
   <221> misc_feature
   <222> (1311)..(1311)
   <223> n is a, c, g, or t
<220>
   <221> misc_feature
   <222> (1320)..(1320)
   <223> n is a, c, g, or t
<220>
   <221> misc_feature
   <222> (1326)..(1326)
   <223> n is a, c, g, or t
<220>
   <221> misc_feature
   <222> (1329)..(1329)
   <223> n is a, c, g, or t
<220>
   <221> misc_feature
   <222> (1332)..(1332)
   <223> n is a, c, g, or t
<220>
   <221> misc_feature
   <222> (1338)..(1338)
   <223> n is a, c, g, or t
<220>
   <221> misc_feature
   <222> (1350)..(1350)
   <223> n is a, c, g, or t
<220>
   <221> misc_feature
   <222> (1353)..(1353)
   <223> n is a, c, g, or t
<220>
   <221> misc_feature
   <222> (1362)..(1362)
   <223> n is a, c, g, or t
<220>
   <221> misc_feature
   <222> (1368)..(1368)
   <223> n is a, c, g, or t
<220>
   <221> misc_feature
   <222> (1374)..(1374)
   <223> n is a, c, g, or t
<220>
   <221> misc_feature
   <222> (1383)..(1383)
   <223> n is a, c, g, or t
<220>
   <221> misc_feature
   <222> (1389)..(1389)
   <223> n is a, c, g, or t
<220>
   <221> misc_feature
   <222> (1392)..(1392)
   <223> n is a, c, g, or t
<220>
   <221> misc_feature
   <222> (1398)..(1398)
   <223> n is a, c, g, or t
<220>
   <221> misc_feature
   <222> (1401)..(1401)
   <223> n is a, c, g, or t
<220>
   <221> misc_feature
   <222> (1407)..(1407)
   <223> n is a, c, g, or t
<220>
   <221> misc_feature
   <222> (1416)..(1416)
   <223> n is a, c, g, or t
<220>
   <221> misc_feature
   <222> (1419)..(1419)
   <223> n is a, c, g, or t
<220>
   <221> misc_feature
   <222> (1422)..(1422)
   <223> n is a, c, g, or t
<220>
   <221> misc_feature
   <222> (1425)..(1425)
   <223> n is a, c, g, or t
<220>
   <221> misc_feature
   <222> (1431)..(1431)
   <223> n is a, c, g, or t
<220>
   <221> misc_feature
   <222> (1437)..(1437)
   <223> n is a, c, g, or t
<220>
   <221> misc_feature
   <222> (1443)..(1443)
   <223> n is a, c, g, or t
<220>
   <221> misc_feature
   <222> (1449)..(1449)
   <223> n is a, c, g, or t
<220>
   <221> misc_feature
   <222> (1452)..(1452)
   <223> n is a, c, g, or t
<220>
   <221> misc_feature
   <222> (1455)..(1455)
   <223> n is a, c, g, or t
<220>
   <221> misc_feature
   <222> (1458)..(1458)
   <223> n is a, c, g, or t
<220>
   <221> misc_feature
   <222> (1461)..(1461)
   <223> n is a, c, g, or t
<220>
   <221> misc_feature
   <222> (1476)..(1476)
   <223> n is a, c, g, or t
<220>
   <221> misc_feature
   <222> (1479)..(1479)
   <223> n is a, c, g, or t
<220>
   <221> misc_feature
   <222> (1482)..(1482)
   <223> n is a, c, g, or t
<220>
   <221> misc_feature
   <222> (1485)..(1485)
   <223> n is a, c, g, or t
<220>
   <221> misc_feature
   <222> (1491)..(1491)
   <223> n is a, c, g, or t
<220>
   <221> misc_feature
   <222> (1497)..(1497)
   <223> n is a, c, g, or t
<220>
   <221> misc_feature
   <222> (1506)..(1506)
   <223> n is a; c, g, or t
<220>
   <221> misc_feature
   <222> (1518)..(1518)
   <223> n is a, c, g, or t
<220>
   <221> misc_feature
   <222> (1521)..(1521)
   <223> n is a, c, g, or t
<220>
   <221> misc_feature
   <222> (1524)..(1524)
   <223> n is a, c, g, or t
<220>
   <221> misc_feature
   <222> (1536)..(1536)
   <223> n is a, c, g, or t
<220>
   <221> misc_feature
   <222> (1545)..(1545)
   <223> n is a, c, g, or t
<220>
   <221> misc_feature
   <222> (1551).. (1551)
   <223> n is a, c, g, or t
<220>
   <221> misc_feature
   <222> (1554)..(1554)
   <223> n is a, c, g, or t
<220>
   <221> misc_feature
   <222> (1563)..(1563)
   <223> n is a, c, g, or t
<220>
   <221> misc_feature
   <222> (1572)..(1572)
   <223> n is a, c, g, or t
<220>
   <221> misc_feature
   <222> (1581)..(1581)
   <223> n is a, c, g, or t
<220>
   <221> misc_feature
   <222> (1587)..(1587)
   <223> n is a, c, g, or t
<220>
   <221> misc_feature
   <222> (1590)..(1590)
   <223> n is a, c, g, or t
<220>
   <221> misc_feature
   <222> (1593)..(1593)
   <223> n is a, c, g, or t
<220>
   <221> misc_feature
   <222> (1596)..(1596)
   <223> n is a, c, g, or t
<220>
   <221> misc_feature
   <222> (1599)..(1599)
   <223> n is a, c, g, or t
<220>
   <221> misc_feature
   <222> (1605)..(1605)
   <223> n is a, c, g, or t
<220>
   <221> misc_feature
   <222> (1623)..(1623)
   <223> n is a, c, g, or t
<220>
   <221> misc_feature
   <222> (1629)..(1629)
   <223> n is a, c, g, or t
<220>
   <221> misc_feature
   <222> (1635)..(1635)
   <223> n is a, c, g, or t
<220>
   <221> misc_feature
   <222> (1641)..(1641)
   <223> n is a, c, g, or t
<220>
   <221> misc_feature
   <222> (1644)..(1644)
   <223> n is a, c, g, or t
<220>
   <221> misc_feature
   <222> (1662)..(1662)
   <223> n is a, c, g, or t
<220>
   <221> misc_feature
   <222> (1668)..(1668)
   <223> n is a, c, g, or t
<220>
   <221> misc_feature
   <222> (1671)..(1671)
   <223> n is a, c, g, or t
<220>
   <221> misc_feature
   <222> (1674)..(1674)
   <223> n is a, c, g, or t
<220>
   <221> misc_feature
   <222> (1677)..(1677)
   <223> n is a, c, g, or t
<220>
   <221> misc_feature
   <222> (1680)..(1680)
   <223> n is a, c, g, or t
<220>
   <221> misc_feature
   <222> (1686)..(1686)
   <223> n is a, c, g, or t
<220>
   <221> misc_feature
   <222> (1692)..(1692)
   <223> n is a, c, g, or t
<220>
   <221> misc_feature
   <222> (1701)..(1701)
   <223> n is a, c, g, or t
<220>
   <221> misc_feature
   <222> (1704)..(1704)
   <223> n is a, c, g, or t
<220>
   <221> misc_feature
   <222> (1722)..(1722)
   <223> n is a, c, g, or t
<220>
   <221> misc_feature
   <222> (1728)..(1728)
   <223> n is a, c, g, or t
<220>
   <221> misc_feature
   <222> (1737)..(1737)
   <223> n is a, c, g, or t
<220>
   <221> misc_feature
   <222> (1749)..(1749)
   <223> n is a, c, g, or t
<220>
   <221> misc_feature
   <222> (1755)..(1755)
   <223> n is a, c, g, or t
<220>
   <221> misc_feature
   <222> (1758)..(1758)
   <223> n is a, c, g, or t
<220>
   <221> misc_feature
   <222> (1764)..(1764)
   <223> n is a, c, g, or t
<220>
   <221> misc_feature
   <222> (1767)..(1767)
   <223> n is a, c, g, or t
<220>
   <221> misc_feature
   <222> (1770)..(1770)
   <223> n is a, c, g, or t
<220>
   <221> misc_feature
   <222> (1776)..(1776)
   <223> n is a, c, g, or t
<220>
   <221> misc_feature
   <222> (1785)..(1785)
   <223> n is a, c, g, or t
<220>
   <221> misc_feature
   <222> (1794)..(1794)
   <223> n is a, c, g, or t
<220>
   <221> misc_feature
   <222> (1803)..(1803)
   <223> n is a, c, g, or t
<220>
   <221> misc_feature
   <222> (1806)..(1806)
   <223> n is a, c, g, or t
<220>
   <221> misc_feature
   <222> (1821)..(1821)
   <223> n is a, c, g, or t
<220>
   <221> misc_feature
   <222> (1824)..(1824)
   <223> n is a, c, g, or t
<220>
   <221> misc_feature
   <222> (1827)..(1827)
   <223> n is a, c, g, or t
<220>
   <221> misc_feature
   <222> (1830)..(1830)
   <223> n is a, c, g, or t
<220>
   <221> misc_feature
   <222> (1836)..(1836)
   <223> n is a, c, g, or t
<220>
   <221> misc_feature
   <222> (1839)..(1839)
   <223> n is a, c, g, or t
<220>
   <221> misc_feature
   <222> (1848)..(1848)
   <223> n is a, c, g, or t
<400> 3
   atgggnwsng gnytnttyaa rccnmgngtn cayccngayy tnmgngaygt nttywsnaar      60
   atgwsnttyt tygayaarat hggnttyytn ttyathcayg cnttygayaa rmgnaayytn     120
   tggcayaarg tnccngtncc nathggnytn ytntayytna ayacnmgnmg nacnytnytn     180
   garaartaya ayytnytngc ngtnggnmgn wsnwsncayg gngcnytntt ygayccnaar     240
   garttyytnt aymgnacnga rgayggnaar tayaaygayc cncayaaygc ngargcnggn     300
   wsncaraaya cnttyttygg nmgnaayatg garccngtng aycarcarga ygarytnatg     360
   wsnccngayc cnttygtngt ngcnacnaar ytnytngcnm gnmgngarta yaargayacn     420
   ggnaarcart tyaayathyt ngcngcngcn tggathcart tyatggtnca ygaytggatg     480
   gaycayatgg argayacngg ncarathggn athacngcnc cnaargargt ngcnaaygar     540
   tgyccnytna arwsnttyaa rttycayccn acnaargary tnccnacnaa ywsngayggn     600
   athaarathg gncaytayaa yathmgnacn gcntggtggg ayggnwsngc ngtntayggn     660
   aayaaygarg armgngcnga raarytnmgn acntaygtng ayggnaaryt ngtnathggn     720
   gaygayggny tnytnytnca yaargaraay ggngtngcny tnwsnggnga yathmgnaay     780
   wsntgggcng gngtnwsnat hytncargcn ytnttygtna argarcayaa ygcngtntgy     840
   gaygcnatha argargarca yccnaayytn wsngaygarg arytntaymg ntaygcnaar     900
   ytngtnacnw sngcngtnat hgcnaargtn cayacnathg aytggacngt ngarytnytn     960
   aaracnaara cnatgmgngc ngcnatgmgn gcnaaytggt ayggnytnyt nggnaaraar    1020
   athaargaya cnttyggnca yathggnggn ccnathytng gnggnytngt nggnytnaar    1080
   aarccnaaya aycayggngt nccntaywsn ytnacngarg arttyacnws ngtntaymgn    1140
   atgcaywsny tnathccnws nacnytnaar ytnmgngayc cnacnggnca rccngaygcn    1200
   aayaaywsnc cnccntgyyt ngargayath gayathggng aratgathgg nytnaarggn    1260
   gargarcary tnwsnaarat hggnttygar aarcargcny tnwsnatggg ntaycargcn    1320
   tgyggngcny tngarytntg gaaytayccn wsnttyttym gnaayytnat hccncaraay    1380
   ytngayggna cnaaymgnws ngaymgnath gayytngcng cnytngargt ntaymgngay    1440
   mgngarmgnw sngtnccnmg ntayaaygar ttymgnmgnm gnytnttyyt nathccnath   1500
   aarwsntggg argayytnac nwsngayaar gaygcnathg aracnathmg ngcnathtay   1560
   ggngaygayg tngaraaryt ngayytnytn gtnggnytna tggcngaraa raarathaar   1620
   ggnttygcna thwsngarac ngcnttyaay athttyathy tnatggcnws nmgnmgnytn   1680
   gargcngaym gnttyttyac nwsnaaytty aaygargara cntayacnaa raarggnatg   1740
   cartgggtna aracnacnga rggnytnmgn gaygtnatha aymgncayta yccngarath   1800
   acngcnaart ggatgaarws nwsnwsngcn ttywsngtnt gggaygcnga ytay         1854
<210> 4
   <211> 1854
   <212> DNA
   <213> Oryza sativa
<400> 4
   atgggcagcg gcctgtttaa accgcgcgtg catccggatc tgcgcgatgt gtttagcaaa     60
   atgagctttt ttgataaaat tggctttctg tttattcatg cgtttgataa acgcaacctg    120
   tggcataaag tgccggtgcc gattggcctg ctgtatctga acacccgccg caccctgctg    180
   gaaaaatata acctgctggc ggtgggccgc agcagccatg gcgcgctgtt tgatccgaaa    240
   gaatttctgt atcgcaccga agatggcaaa tataacgatc cgcataacgc ggaagcgggc    300
   agccagaaca ccttttttgg ccgcaacatg gaaccggtgg atcagcagga tgaactgatg    360
   agcccggatc cgtttgtggt ggcgaccaaa ctgctggcgc gccgcgaata taaagatacc    420
   ggcaaacagt ttaacattct ggcggcggcg tggattcagt ttatggtgca tgattggatg    480
   gatcatatgg aagataccgg ccagattggc attaccgcgc cgaaagaagt ggcgaacgaa    540
   tgcccgctga aaagctttaa atttcatccg accaaagaac tgccgaccaa cagcgatggc    600
   attaaaattg gccattataa cattcgcacc gcgtggtggg atggcagcgc ggtgtatggc    660
   aacaacgaag aacgcgcgga aaaactgcgc acctatgtgg atggcaaact ggtgattggc    720
   gatgatggcc tgctgctgca taaagaaaac ggcgtggcgc tgagcggcga tattcgcaac    780
   agctgggcgg gcgtgagcat tctgcaggcg ctgtttgtga aagaacataa cgcggtgtgc    840
   gatgcgatta aagaagaaca tccgaacctg agcgatgaag aactgtatcg ctatgcgaaa    900
   ctggtgacca gcgcggtgat tgcgaaagtg cataccattg attggaccgt ggaactgctg    960
   aaaaccaaaa ccatgcgcgc ggcgatgcgc gcgaactggt atggcctgct gggcaaaaaa   1020
   attaaagata cctttggcca tattggcggc ccgattctgg gcggcctggt gggcctgaaa   1080
   aaaccgaaca accatggcgt gccgtatagc ctgaccgaag aatttaccag cgtgtatcgc   1140
   atgcatagcc tgattccgag caccctgaaa ctgcgcgatc cgaccggcca gccggatgcg   1200
   aacaacagcc cgccgtgcct ggaagatatt gatattggcg aaatgattgg cctgaaaggc   1260
   gaagaacagc tgagcaaaat tggctttgaa aaacaggcgc tgagcatggg ctatcaggcg   1320
   tgcggcgcgc tggaactgtg gaactatccg agcttttttc gcaacctgat tccgcagaac   1380
   ctggatggca ccaaccgcag cgatcgcatt gatctggcgg cgctggaagt gtatcgcgat   1440
   cgcgaacgca gcgtgccgcg ctataacgaa tttcgccgcc gcctgtttct gattccgatt   1500
   aaaagctggg aagatctgac cagcgataaa gatgcgattg aaaccattcg cgcgatttat   1560
   ggcgatgatg tggaaaaact ggatctgctg gtgggcctga tggcggaaaa aaaaattaaa   1620
   ggctttgcga ttagcgaaac cgcgtttaac atttttattc tgatggcgag ccgccgcctg   1680
   gaagcggatc gcttttttac cagcaacttt aacgaagaaa cctataccaa aaaaggcatg   1740
   cagtgggtga aaaccaccga aggcctgcgc gatgtgatta accgccatta tccggaaatt   1800
   accgcgaaat ggatgaaaag cagcagcgcg tttagcgtgt gggatgcgga ttat         1854

### SEQUENCE LISTING

<110> Symrise AG
<120> Aldehydherstellung aus Fettsäuren
<130> SA 7322-03WO
<160> 4
<170> PatentIn version 3.3
<210> 1
   <211> 231
   <212> PRT
   <213> Oryza sativa
<400> 1
   Gly Pro Ile Leu Gly Gly Leu Val Gly Leu Lys Lys Pro Asn Asn His
   1               5                   10                  15
   Gly Val Pro Tyr Ser Leu Thr Glu Glu Phe Thr Ser Val Tyr Arg Met
               20                  25                  30
   His Ser Leu Ile Pro Ser Thr Leu Lys Leu Arg Asp Pro Thr Gly Gln
           35                  40                  45
   Pro Asp Ala Asn Asn Ser Pro Pro Cys Leu Glu Asp Ile Asp Ile Gly
       50                  55                  60
   Glu Met Ile Gly Leu Lys Gly Glu Glu Gln Leu Ser Lys Ile Gly Phe
   65                  70                  75                  80
   Glu Lys Gln Ala Leu Ser Met Gly Tyr Gln Ala Cys Gly Ala Leu Glu
                   85                  90                  95
   Leu Trp Asn Tyr Pro Ser Phe Phe Arg Asn Leu Ile Pro Gln Asn Leu
               100                 105                 110
   Asp Gly Thr Asn Arg Ser Asp Arg Ile Asp Leu Ala Ala Leu Glu Val
           115                 120                 125
   Tyr Arg Asp Arg Glu Arg Ser Val Pro Arg Tyr Asn Glu Phe Arg Arg
       130                 135                 140
   Arg Leu Phe Leu Ile Pro Ile Lys Ser Trp Glu Asp Leu Thr Ser Asp
   145                 150                 155                 160
   Lys Asp Ala Ile Glu Thr Ile Arg Ala Ile Tyr Gly Asp Asp Val Glu
                   165                 170                 175
   Lys Leu Asp Leu Leu Val Gly Leu Met Ala Glu Lys Lys Ile Lys Gly
               180                 185                 190
   Phe Ala Ile Ser Glu Thr Ala Phe Asn Ile Phe Ile Leu Met Ala Ser
           195                 200                 205
   Arg Arg Leu Glu Ala Asp Arg Phe Phe Thr Ser Asn Phe Asn Glu Glu
       210                 215                 220
   Thr Tyr Thr Lys Lys Gly Met
   225                 230
<210> 2
   <211> 618
   <212> PRT
   <213> Oryza sativa
<400> 2
   Met Gly Ser Gly Leu Phe Lys Pro Arg Val His Pro Asp Leu Arg Asp
   1               5                   10                  15
   Val Phe Ser Lys Met Ser Phe Phe Asp Lys Ile Gly Phe Leu Phe Ile
               20                  25                  30
   His Ala Phe Asp Lys Arg Asn Leu Trp His Lys Val Pro Val Pro Ile
           35                  40                  45
   Gly Leu Leu Tyr Leu Asn Thr Arg Arg Thr Leu Leu Glu Lys Tyr Asn
       50                  55                  60
   Leu Leu Ala Val Gly Arg Ser Ser His Gly Ala Leu Phe Asp Pro Lys
   65                  70                  75                  80
   Glu Phe Leu Tyr Arg Thr Glu Asp Gly Lys Tyr Asn Asp Pro His Asn
                   85                  90                  95
   Ala Glu Ala Gly Ser Gln Asn Thr Phe Phe Gly Arg Asn Met Glu Pro
               100                 105                 110
   Val Asp Gln Gln Asp Glu Leu Met Ser Pro Asp Pro Phe Val Val Ala
           115                 120                 125
   Thr Lys Leu Leu Ala Arg Arg Glu Tyr Lys Asp Thr Gly Lys Gln Phe
       130                 135                 140
   Asn Ile Leu Ala Ala Ala Trp Ile Gln Phe Met Val His Asp Trp Met
   145                 150                 155                 160
   Asp His Met Glu Asp Thr Gly Gln Ile Gly Ile Thr Ala Pro Lys Glu
                   165                 170                 175
   Val Ala Asn Glu Cys Pro Leu Lys Ser Phe Lys Phe His Pro Thr Lys
               180                 185                 190
   Glu Leu Pro Thr Asn Ser Asp Gly Ile Lys Ile Gly His Tyr Asn Ile
           195                 200                 205
   Arg Thr Ala Trp Trp Asp Gly Ser Ala Val Tyr Gly Asn Asn Glu Glu
       210                 215                 220
   Arg Ala Glu Lys Leu Arg Thr Tyr Val Asp Gly Lys Leu Val Ile Gly
   225                 230                 235                 240
   Asp Asp Gly Leu Leu Leu His Lys Glu Asn Gly Val Ala Leu Ser Gly
                   245                 250                 255
   Asp Ile Arg Asn Ser Trp Ala Gly Val Ser Ile Leu Gln Ala Leu Phe
               260                 265                 270
   Val Lys Glu His Asn Ala Val Cys Asp Ala Ile Lys Glu Glu His Pro
           275                 280                 285
   Asn Leu Ser Asp Glu Glu Leu Tyr Arg Tyr Ala Lys Leu Val Thr Ser
       290                 295                 300
   Ala Val Ile Ala Lys Val His Thr Ile Asp Trp Thr Val Glu Leu Leu
   305                 310                 315                 320
   Lys Thr Lys Thr Met Arg Ala Ala Met Arg Ala Asn Trp Tyr Gly Leu
                   325                 330                 335
   Leu Gly Lys Lys Ile Lys Asp Thr Phe Gly His Ile Gly Gly Pro Ile
               340                 345                 350
   Leu Gly Gly Leu Val Gly Leu Lys Lys Pro Asn Asn His Gly Val Pro
           355                 360                 365
   Tyr Ser Leu Thr Glu Glu Phe Thr Ser Val Tyr Arg Met His Ser Leu
       370                 375                 380
   Ile Pro Ser Thr Leu Lys Leu Arg Asp Pro Thr Gly Gln Pro Asp Ala
   385                 390                 395                 400
   Asn Asn Ser Pro Pro Cys Leu Glu Asp Ile Asp Ile Gly Glu Met Ile
                   405                 410                 415
   Gly Leu Lys Gly Glu Glu Gln Leu Ser Lys Ile Gly Phe Glu Lys Gln
   420 425 430
   Ala Leu Ser Met Gly Tyr Gln Ala Cys Gly Ala Leu Glu Leu Trp Asn
   435 440 445
   Tyr Pro Ser Phe Phe Arg Asn Leu Ile Pro Gln Asn Leu Asp Gly Thr
   450 455 460
   Asn Arg Ser Asp Arg Ile Asp Leu Ala Ala Leu Glu Val Tyr Arg Asp
   465 470 475 480
   Arg Glu Arg Ser Val Pro Arg Tyr Asn Glu Phe Arg Arg Arg Leu Phe
   485 490 495
   Leu Ile Pro Ile Lys Ser Trp Glu Asp Leu Thr Ser Asp Lys Asp Ala
   500 505 510
   Ile Glu Thr Ile Arg Ala Ile Tyr Gly Asp Asp Val Glu Lys Leu Asp
   515 520 525
   Leu Leu Val Gly Leu Met Ala Glu Lys Lys Ile Lys Gly Phe Ala Ile
   530 535 540
   Ser Glu Thr Ala Phe Asn Ile Phe Ile Leu Met Ala Ser Arg Arg Leu
   545 550 555 560
   Glu Ala Asp Arg Phe Phe Thr Ser Asn Phe Asn Glu Glu Thr Tyr Thr
   565 570 575
   Lys Lys Gly Met Gln Trp Val Lys Thr Thr Glu Gly Leu Arg Asp Val
   580 585 590
   Ile Asn Arg His Tyr Pro Glu Ile Thr Ala Lys Trp Met Lys Ser Ser
   595 600 605
   Ser Ala Phe Ser Val Trp Asp Ala Asp Tyr
   610 615
<210> 3
   <211> 1854
   <212> DNA
   <213> Oryza sativa
<220>
   <221> misc_feature
   <222> (6)..(6)
   <223> n is a, c, g, or t
<220>
   <221> misc_feature
   <222> (9)..(9)
   <223> n is a, c, g, or t
<220>
   <221> misc_feature
   <222> (12)..(12)
   <223> n is a, c, g, or t
<220>
   <221> misc_feature
   <222> (15)..(15)
   <223> n is a, c, g, or t
<220>
   <221> misc_feature
   <222> (24)..(24)
   <223> n is a, c, g, or t
<220>
   <221> misc_feature
   <222> (27)..(27)
   <223> n is a, c, g, or t
<220>
   <221> misc_feature
   <222> (30)..(30)
   <223> n is a, c, g, or t
<220>
   <221> misc_feature
   <222> (36)..(36)
   <223> n is a, c, g, or t
<220>
   <221> misc_feature
   <222> (42)..(42)
   <223> n is a, c, g, or t
<220>
   <221> misc_feature
   <222> (45)..(45)
   <223> n is a, c, g, or t
<220>
   <221> misc_feature
   <222> (51)..(51)
   <223> n is a, c, g, or t
<220>
   <221> misc_feature
   <222> (57)..(57)
   <223> n is a, c, g, or t
<220>
   <221> misc_feature
   <222> (66)..(66)
   <223> n is a, c, g, or t
<220>
   <221> misc_feature
   <222> (84)..(84)
   <223> n is a, c, g, or t
<220>
   <221> misc_feature
   <222> (90)..(90)
   <223> n is a, c, g, or t
<220>
   <221> misc_feature
   <222> (102)..(102)
   <223> n is a, c, g, or t
<220>
   <221> misc_feature
   <222> (114)..(114)
   <223> n is a, c, g, or t
<220>
   <221> misc_feature
   <222> (120)..(120)
   <223> n is a, c, g, or t
<220>
   <221> misc_feature
   <222> (132)..(132)
   <223> n is a, c, g, or t
<220>
   <221> misc_feature
   <222> (135)..(135)
   <223> n is a, c, g, or t
<220>
   <221> misc_feature
   <222> (138)..(138)
   <223> n is a, c, g, or t
<220>
   <221> misc_feature
   <222> (141)..(141)
   <223> n is a, c, g, or t
<220>
   <221> misc_feature
   <222> (147)..(147)
   <223> n is a, c, g, or t
<220>
   <221> misc_feature
   <222> (150)..(150)
   <223> n is a, c, g, or t
<220>
   <221> misc_feature
   <222> (153)..(153)
   <223> n is a, c, g, or t
<220>
   <221> misc_feature
   <222> (159)..(159)
   <223> n is a, c, g, or t
<220>
   <221> misc_feature
   <222> (165)..(165)
   <223> n is a, c, g, or t
<220>
   <221> misc_feature
   <222> (168)..(168)
   <223> n is a, c, g, or t
<220>
   <221> misc_feature
   <222> (171)..(171)
   <223> n is a, c, g, or t
<220>
   <221> misc_feature
   <222> (174)..(174)
   <223> n is a, c, g, or t
<220>
   <221> misc_feature
   <222> (177)..(177)
   <223> n is a, c, g, or t
<220>
   <221> misc_feature
   <222> (180)..(180)
   <223> n is a, c, g, or t
<220>
   <221> misc_feature
   <222> (195)..(195)
   <223> n is a, c, g, or t
<220>
   <221> misc_feature
   <222> (198)..(198)
   <223> n is a, c, g, or t
<220>
   <221> misc_feature
   <222> (201)..(201)
   <223> n is a, c, g, or t
<220>
   <221> misc_feature
   <222> (204)..(204)
   <223> n is a, c, g, or t
<220>
   <221> misc_feature
   <222> (207)..(207)
   <223> n is a, c, g, or t
<220>
   <221> misc_feature
   <222> (210)..(210)
   <223> n is a, c, g, or t
<220>
   <221> misc_feature
   <222> (213)..(213)
   <223> n is a, c, g, or t
<220>
   <221> misc_feature
   <222> (216)..(216)
   <223> n is a, c, g, or t
<220>
   <221> misc_feature
   <222> (222)..(222)
   <223> n is a, c, g, or t
<220>
   <221> misc_feature
   <222> (225)..(225)
   <223> n is a, c, g, or t
<220>
   <221> misc_feature
   <222> (228)..(228)
   <223> n is a, c, g, or t
<220>
   <221> misc_feature
   <222> (237)..(237)
   <223> n is a, c, g, or t
<220>
   <221> misc_feature
   <222> (249)..(249)
   <223> n is a, c, g, or t
<220>
   <221> misc_feature
   <222> (255)..(255)
   <223> n is a, c, g, or t
<220>
   <221> misc_feature
   <222> (258)..(258)
   <223> n is a, c, g, or t
<220>
   <221> misc_feature
   <222> (267)..(267)
   <223> n is a, c, g, or t
<220>
   <221> misc_feature
   <222> (282)..(282)
   <223> n is a, c, g, or t
<220>
   <221> misc_feature
   <222> (291)..(291)
   <223> n is a, c, g, or t
<220>
   <221> misc_feature
   <222> (297)..(297)
   <223> n is a, c, g, or t
<220>
   <221> misc_feature
   <222> (300)..(300)
   <223> n is a, c, g, or t
<220>
   <221> misc_feature
   <222> (303)..(303)
   <223> n is a, c, g, or t
<220>
   <221> misc_feature
   <222> (312)..(312)
   <223> n is a, c, g, or t
<220>
   <221> misc_feature
   <222> (321)..(321)
   <223> n is a, c, g, or t
<220>
   <221> misc_feature
   <222> (324)..(324)
   <223> n is a, c, g, or t
<220>
   <221> misc_feature
   <222> (336)..(336)
   <223> n is a, c, g, or t
<220>
   <221> misc_feature
   <222> (339)..(339)
   <223> n is a, c, g, or t
<220>
   <221> misc_feature
   <222> (357)..(357)
   <223> n is a, c, g, or t
<220>
   <221> misc_feature
   <222> (363)..(363)
   <223> n is a, c, g, or t
<220>
   <221> misc_feature
   <222> (366)..(366)
   <223> n is a, c, g, or t
<220>
   <221> misc_feature
   <222> (372)..(372)
   <223> n is a, c, g, or t
<220>
   <221> misc_feature
   <222> (378)..(378)
   <223> n is a, c, g, or t
<220>
   <221> misc_feature
   <222> (381)..(381)
   <223> n is a, c, g, or t
<220>
   <221> misc_feature
   <222> (384)..(384)
   <223> n is a, c, g, or t
<220>
   <221> misc_feature
   <222> (387)..(387)
   <223> n is a, c, g, or t
<220>
   <221> misc_feature
   <222> (393)..(393)
   <223> n is a, c, g, or t
<220>
   <221> misc_feature
   <222> (396)..(396)
   <223> n is a, c, g, or t
<220>
   <221> misc_feature
   <222> (399)..(399)
   <223> n is a, c, g, or t
<220>
   <221> misc_feature
   <222> (402)..(402)
   <223> n is a, c, g, or t
<220>
   <221> misc_feature
   <222> (405)..(405)
   <223> n is a, c, g, or t
<220>
   <221> misc_feature
   <222> (420)..(420)
   <223> n is a, c, g, or t
<220>
   <221> misc_feature
   <222> (423)..(423)
   <223> n is a, c, g, or t
<220>
   <221> misc_feature
   <222> (441)..(441)
   <223> n is a, c, g, or t
<220>
   <221> misc_feature
   <222> (444)..(444)
   <223> n is a, c, g, or t
<220>
   <221> misc_feature
   <222> (447)..(447)
   <223> n is a, c, g, or t
<220>
   <221> misc_feature
   <222> (450)..(450)
   <223> n is a, c, g, or t
<220>
   <221> misc_feature
   <222> (468)..(468)
   <223> n is a, c, g, or t
<220>
   <221> misc_feature
   <222> (498)..(498)
   <223> n is a, c, g, or t
<220>
   <221> misc_feature
   <222> (501)..(501)
   <223> n is a, c, g, or t
<220>
   <221> misc_feature
   <222> (510)..(510)
   <223> n is a, c, g, or t
<220>
   <221> misc_feature
   <222> (516)..(516)
   <223> n is a, c, g, or t
<220>
   <221> misc_feature
   <222> (519)..(519)
   <223> n is a, c, g, or t
<220>
   <221> misc_feature
   <222> (522)..(522)
   <223> n is a, c, g, or t
<220>
   <221> misc_feature
   <222> (531)..(531)
   <223> n is a, c, g, or t
<220>
   <221> misc_feature
   <222> (534)..(534)
   <223> n is a, c, g, or t
<220>
   <221> misc_feature
   <222> (546)..(546)
   <223> n is a, c, g, or t
<220>
   <221> misc_feature
   <222> (549)..(549)
   <223> n is a, c, g, or t
<220>
   <221> misc_feature
   <222> (555)..(555)
   <223> n is a, c, g, or t
<220>
   <221> misc_feature
   <222> (570)..(570)
   <223> n is a, c, g, or t
<220>
   <221> misc_feature
   <222> (573)..(573)
   <223> n is a, c, g, or t
<220>
   <221> misc_feature
   <222> (582)..(582)
   <223> n is a, c, g, or t
<220>
   <221> misc_feature
   <222> (585)..(585)
   <223> n is a, c, g, or t
<220>
   <221> misc_feature
   <222> (588)..(588)
   <223> n is a, c, g, or t
<220>
   <221> misc_feature
   <222> (594)..(594)
   <223> n is a, c, g, or t
<220>
   <221> misc_feature
   <222> (600)..(600)
   <223> n is a, c, g, or t
<220>
   <221> misc_feature
   <222> (612)..(612)
   <223> n is a, c, g, or t
<220>
   <221> misc_feature
   <222> (627)..(627)
   <223> n is a, c, g, or t
<220>
   <221> misc_feature
   <222> (630)..(630)
   <223> n is a, c, g, or t
<220>
   <221> misc_feature
   <222> (633)..(633)
   <223> n is a, c, g, or t
<220>
   <221> misc_feature
   <222> (645)..(645)
   <223> n is a, c, g, or t
<220>
   <221> misc_feature
   <222> (648)..(648)
   <223> n is a, c, g, or t
<220>
   <221> misc_feature
   <222> (651)..(651)
   <223> n is a, c, g, or t
<220>
   <221> misc_feature
   <222> (654)..(654)
   <223> n is a, c, g, or t
<220>
   <221> misc_feature
   <222> (660)..(660)
   <223> n is a, c, g, or t
<220>
   <221> misc_feature
   <222> (675)..(675)
   <223> n is a, c, g, or t
<220>
   <221> misc_feature
   <222> (678)..(678)
   <223> n is a, c, g, or t
<220>
   <221> misc_feature
   <222> (687)..(687)
   <223> n is a, c, g, or t
<220>
   <221> misc_feature
   <222> (690)..(690)
   <223> n is a, c, g, or t
<220>
   <221> misc_feature
   <222> (693)..(693)
   <223> n is a, c, g, or t
<220>
   <221> misc_feature
   <222> (699)..(699)
   <223> n is a, c, g, or t
<220>
   <221> misc_feature
   <222> (705)..(705)
   <223> n is a, c, g, or t
<220>
   <221> misc_feature
   <222> (711)..(711)
   <223> n is a, c, g, or t
<220>
   <221> misc_feature
   <222> (714)..(714)
   <223> n is a, c, g, or t
<220>
   <221> misc_feature
   <222> (720)..(720)
   <223> n is a, c, g, or t
<220>
   <221> misc_feature
   <222> (729)..(729)
   <223> n is a, c, g, or t
<220>
   <221> misc_feature
   <222> (732)..(732)
   <223> n is a, c, g, or t
<220>
   <221> misc_feature
   <222> (735)..(735)
   <223> n is a, c, g, or t
<220>
   <221> misc_feature
   <222> (738)..(738)
   <223> n is a, c, g, or t
<220>
   <221> misc_feature
   <222> (753)..(753)
   <223> n is a, c, g, or t
<220>
   <221> misc_feature
   <222> (756)..(756)
   <223> n is a, c, g, or t
<220>
   <221> misc_feature
   <222> (759)..(759)
   <223> n is a, c, g, or t
<220>
   <221> misc_feature
   <222> (762)..(762)
   <223> n is a, c, g, or t
<220>
   <221> misc_feature
   <222> (765)..(765)
   <223> n is a, c, g, or t
<220>
   <221> misc_feature
   <222> (768)..(768)
   <223> n is a, c, g, or t
<220>
   <221> misc_feature
   <222> (777)..(777)
   <223> n is a, c, g, or t
<220>
   <221> misc_feature
   <222> (783)..(783)
   <223> n is a, c, g, or t
<220>
   <221> misc_feature
   <222> (789)..(789)
   <223> n is a, c, g, or t
<220>
   <221> misc_feature
   <222> (792)..(792)
   <223> n is a, c, g, or t
<220>
   <221> misc_feature
   <222> (795)..(795)
   <223> n is a, c, g, or t
<220>
   <221> misc_feature
   <222> (798)..(798)
   <223> n is a, c, g, or t
<220>
   <221> misc_feature
   <222> (804)..(804)
   <223> n is a, c, g, or t
<220>
   <221> misc_feature
   <222> (810)..(810)
   <223> n is a, c, g, or t
<220>
   <221> misc_feature
   <222> (813)..(813)
   <223> n is a, c, g, or t
<220>
   <221> misc_feature
   <222> (819)..(819)
   <223> n is a, c, g, or t
<220>
   <221> misc_feature
   <222> (834)..(834)
   <223> n is a, c, g, or t
<220>
   <221> misc_feature
   <222> (837)..(837)
   <223> n is a, c, g, or t
<220>
   <221> misc_feature
   <222> (846)..(846)
   <223> n is a, c, g, or t
<220>
   <221> misc_feature
   <222> (864)..(864)
   <223> n is a, c, g, or t
<220>
   <221> misc_feature
   <222> (870)..(870)
   <223> n is a, c, g, or t
<220>
   <221> misc_feature
   <222> (873)..(873)
   <223> n is a, c, g, or t
<220>
   <221> misc_feature
   <222> (885)..(885)
   <223> n is a, c, g, or t
<220>
   <221> misc_feature
   <222> (891)..(891)
   <223> n is a, c, g, or t
<220>
   <221> misc_feature
   <222> (897)..(897)
   <223> n is a, c, g, or t
<220>
   <221> misc_feature
   <222> (903)..(903)
   <223> n is a, c, g, or t
<220>
   <221> misc_feature
   <222> (906)..(906)
   <223> n is a, c, g, or t
<220>
   <221> misc_feature
   <222> (909)..(909)
   <223> n is a, c, g, or t
<220>
   <221> misc_feature
   <222> (912)..(912)
   <223> n is a, c, g, or t
<220>
   <221> misc_feature
   <222> (915)..(915)
   <223> n is a, c, g, or t
<220>
   <221> misc_feature
   <222> (918)..(918)
   <223> n is a, c, g, or t
<220>
   <221> misc_feature
   <222> (924)..(924)
   <223> n is a, c, g, or t
<220>
   <221> misc_feature
   <222> (930)..(930)
   <223> n is a, c, g, or t
<220>
   <221> misc_feature
   <222> (936)..(936)
   <223> n is a, c, g, or t
<220>
   <221> misc_feature
   <222> (948)..(948)
   <223> n is a, c, g, or t
<220>
   <221> misc_feature
   <222> (951)..(951)
   <223> n is a, c, g, or t
<220>
   <221> misc_feature
   <222> (957)..(957)
   <223> n is a, c, g, or t
<220>
   <221> misc_feature
   <222> (960)..(960)
   <223> n is a, c, g, or t
<220>
   <221> misc_feature
   <222> (966)..(966)
   <223> n is a, c, g, or t
<220>
   <221> misc_feature
   <222> (972)..(972)
   <223> n is a, c, g, or t
<220>
   <221> misc_feature
   <222> (978)..(978)
   <223> n is a, c, g, or t
<220>
   <221> misc_feature
   <222> (981)..(981)
   <223> n is a, c, g, or t
<220>
   <221> misc_feature
   <222> (984)..(984)
   <223> n is a, c, g, or t
<220>
   <221> misc_feature
   <222> (990)..(990)
   <223> n is a, c, g, or t
<220>
   <221> misc_feature
   <222> (993)..(993)
   <223> n is a, c, g, or t
<220>
   <221> misc_feature
   <222> (1005)..(1005)
   <223> n is a, c, g, or t
<220>
   <221> misc_feature
   <222> (1008)..(1008)
   <223> n is a, c, g, or t
<220>
   <221> misc_feature
   <222> (1011)..(1011)
   <223> n is a, c, g, or t
<220>
   <221> misc_feature
   <222> (1014)..(1014)
   <223> n is a, c, g, or t
<220>
   <221> misc_feature
   <222> (1032)..(1032)
   <223> n is a, c, g, or t
<220>
   <221> misc_feature
   <222> (1038)..(1038)
   <223> n is a, c, g, or t
<220>
   <221> misc_feature
   <222> (1047)..(1047)
   <223> n is a, c, g, or t
<220>
   <221> misc_feature
   <222> (1050)..(1050)
   <223> n is a, c, g, or t
<220>
   <221> misc_feature
   <222> (1053)..(1053)
   <223> n is a, c, g, or t
<220>
   <221> misc_feature
   <222> (1059)..(1059)
   <223> n is a, c, g, or t
<220>
   <221> misc_feature
   <222> (1062)..(1062)
   <223> n is a, c, g, or t
<220>
   <221> misc_feature
   <222> (1065)..(1065)
   <223> n is a, c, g, or t
<220>
   <221> misc_feature
   <222> (1068)..(1068)
   <223> n is a, c, g, or t
<220>
   <221> misc_feature
   <222> (1071)..(1071)
   <223> n is a, c, g, or t
<220>
   <221> misc_feature
   <222> (1074)..(1074)
   <223> n is a, c, g, or t
<220>
   <221> misc_feature
   <222> (1077)..(1077)
   <223> n is a, c, g, or t
<220>
   <221> misc_feature
   <222> (1086)..(1086)
   <223> n is a, c, g, or t
<220>
   <221> misc_feature
   <222> (1098)..(1098)
   <223> n is a, c, g, or t
<220>
   <221> misc_feature
   <222> (1101)..(1101)
   <223> n is a, c, g, or t
<220>
   <221> misc_feature
   <222> (1104)..(1104)
   <223> n is a, c, g, or t
<220>
   <221> misc_feature
   <222> (1110)..(1110)
   <223> n is a, c, g, or t
<220>
   <221> misc_feature
   <222> (1113)..(1113)
   <223> n is a, c, g, or t
<220>
   <221> misc_feature
   <222> (1116)..(1116)
   <223> n is a, c, g, or t
<220>
   <221> misc_feature
   <222> (1128)..(1128)
   <223> n is a, c, g, or t
<220>
   <221> misc_feature
   <222> (1131)..(1131)
   <223> n is a, c, g, or t
<220>
   <221> misc_feature
   <222> (1134)..(1134)
   <223> n is a, c, g, or t
<220>
   <221> misc_feature
   <222> (1140)..(1140)
   <223> n is a, c, g, or t
<220>
   <221> misc_feature
   <222> (1149)..(1149)
   <223> n is a, c, g, or t
<220>
   <221> misc_feature
   <222> (1152)..(1152)
   <223> n is a, c, g, or t
<220>
   <221> misc_feature
   <222> (1158)..(1158)
   <223> n is a, c, g, or t
<220>
   <221> misc_feature
   <222> (1161)..(1161)
   <223> n is a, c, g, or t
<220>
   <221> misc_feature
   <222> (1164)..(1164)
   <223> n is a, c, g, or t
<220>
   <221> misc_feature
   <222> (1167)..(1167)
   <223> n is a, c, g, or t
<220>
   <221> misc_feature
   <222> (1173)..(1173)
   <223> n is a, c, g, or t
<220>
   <221> misc_feature
   <222> (1176)..(1176)
   <223> n is a, c, g, or t
<220>
   <221> misc_feature
   <222> (1182)..(1182)
   <223> n is a, c, g, or t
<220>
   <221> misc_feature
   <222> (1185)..(1185)
   <223> n is a, c, g, or t
<220>
   <221> misc_feature
   <222> (1188)..(1188)
   <223> n is a, c, g, or t
<220>
   <221> misc_feature
   <222> (1194)..(1194)
   <223> n is a, c, g, or t
<220>
   <221> misc_feature
   <222> (1200)..(1200)
   <223> n is a, c, g, or t
<220>
   <221> misc_feature
   <222> (1209)..(1209)
   <223> n is a, c, g, or t
<220>
   <221> misc_feature
   <222> (1212)..(1212)
   <223> n is a, c, g, or t
<220>
   <221> misc_feature
   <222> (1215)..(1215)
   <223> n is a, c, g, or t
<220>
   <221> misc_feature
   <222> (1221)..(1221)
   <223> n is a, c, g, or t
<220>
   <221> misc_feature
   <222> (1239)..(1239)
   <223> n is a, c, g, or t
<220>
   <221> misc_feature
   <222> (1251)..(1251)
   <223> n is a, c, g, or t
<220>
   <221> misc_feature
   <222> (1254)..(1254)
   <223> n is a, c, g, or t
<220>
   <221> misc_feature
   <222> (1260)..(1260)
   <223> n is a, c, g, or t
<220>
   <221> misc_feature
   <222> (1272)..(1272)
   <223> n is a, c, g, or t
<220>
   <221> misc_feature
   <222> (1275)..(1275)
   <223> n is a, c, g, or t
<220>
   <221> misc_feature
   <222> (1284)..(1284)
   <223> n is a, c, g, or t
<220>
   <221> misc_feature
   <222> (1299)..(1299)
   <223> n is a, c, g, or t
<220>
   <221> misc_feature
   <222> (1302)..(1302)
   <223> n is a, c, g, or t
<220>
   <221> misc_feature
   <222> (1305)..(1305)
   <223> n is a, c, g, or t
<220>
   <221> misc_feature
   <222> (1311)..(1311)
   <223> n is a, c, g, or t
<220>
   <221> misc_feature
   <222> (1320)..(1320)
   <223> n is a, c, g, or t
<220>
   <221> misc_feature
   <222> (1326)..(1326)
   <223> n is a, c, g, or t
<220>
   <221> misc_feature
   <222> (1329)..(1329)
   <223> n is a, c, g, or t
<220>
   <221> misc_feature
   <222> (1332)..(1332)
   <223> n is a, c, g, or t
<220>
   <221> misc_feature
   <222> (1338)..(1338)
   <223> n is a, c, g, or t
<220>
   <221> misc_feature
   <222> (1350)..(1350)
   <223> n is a, c, g, or t
<220>
   <221> misc_feature
   <222> (1353)..(1353)
   <223> n is a, c, g, or t
<220>
   <221> misc_feature
   <222> (1362)..(1362)
   <223> n is a, c, g, or t
<220>
   <221> misc_feature
   <222> (1368)..(1368)
   <223> n is a, c, g, or t
<220>
   <221> misc_feature
   <222> (1374)..(1374)
   <223> n is a, c, g, or t
<220>
   <221> misc_feature
   <222> (1383)..(1383)
   <223> n is a, c, g, or t
<220>
   <221> misc_feature
   <222> (1389)..(1389)
   <223> n is a, c, g, or t
<220>
   <221> misc_feature
   <222> (1392)..(1392)
   <223> n is a, c, g, or t
<220>
   <221> misc_feature
   <222> (1398)..(1398)
   <223> n is a, c, g, or t
<220>
   <221> misc_feature
   <222> (1401)..(1401)
   <223> n is a, c, g, or t
<220>
   <221> misc_feature
   <222> (1407)..(1407)
   <223> n is a, c, g, or t
<220>
   <221> misc_feature
   <222> (1416)..(1416)
   <223> n is a, c, g, or t
<220>
   <221> misc_feature
   <222> (1419)..(1419)
   <223> n is a, c, g, or t
<220>
   <221> misc_feature
   <222> (1422)..(1422)
   <223> n is a, c, g, or t
<220>
   <221> misc_feature
   <222> (1425)..(1425)
   <223> n is a, c, g, or t
<220>
   <221> misc_feature
   <222> (1431)..(1431)
   <223> n is a, c, g, or t
<220>
   <221> misc_feature
   <222> (1437)..(1437)
   <223> n is a, c, g, or t
<220>
   <221> misc_feature
   <222> (1443)..(1443)
   <223> n is a, c, g, or t
<220>
   <221> misc_feature
   <222> (1449)..(1449)
   <223> n is a, c, g, or t
<220>
   <221> misc_feature
   <222> (1452)..(1452)
   <223> n is a, c, g, or t
<220>
   <221> misc_feature
   <222> (1455)..(1455)
   <223> n is a, c, g, or t
<220>
   <221> misc_feature
   <222> (1458)..(1458)
   <223> n is a, c, g, or t
<220>
   <221> misc_feature
   <222> (1461)..(1461)
   <223> n is a, c, g, or t
<220>
   <221> misc_feature
   <222> (1476)..(1476)
   <223> n is a, c, g, or t
<220>
   <221> misc_feature
   <222> (1479)..(1479)
   <223> n is a, c, g, or t
<220>
   <221> misc_feature
   <222> (1482)..(1482)
   <223> n is a, c, g, or t
<220>
   <221> misc_feature
   <222> (1485)..(1485)
   <223> n is a, c, g, or t
<220>
   <221> misc_feature
   <222> (1491)..(1491)
   <223> n is a, c, g, or t
<220>
   <221> misc_feature
   <222> (1497)..(1497)
   <223> n is a, c, g, or t
<220>
   <221> misc_feature
   <222> (1506)..(1506)
   <223> n is a, c, g, or t
<220>
   <221> misc_feature
   <222> (1518)..(1518)
   <223> n is a, c, g, or t
<220>
   <221> misc_feature
   <222> (1521)..(1521)
   <223> n is a, c, g, or t
<220>
   <221> misc_feature
   <222> (1524)..(1524)
   <223> n is a, c, g, or t
<220>
   <221> misc_feature
   <222> (1536)..(1536)
   <223> n is a, c, g, or t
<220>
   <221> misc_feature
   <222> (1545)..(1545)
   <223> n is a, c, g, or t
<220>
   <221> misc_feature
   <222> (1551)..(1551)
   <223> n is a, c, g, or t
<220>
   <221> misc_feature
   <222> (1554)..(1554)
   <223> n is a, c, g, or t
<220>
   <221> misc_feature
   <222> (1563)..(1563)
   <223> n is a, c, g, or t
<220>
   <221> misc_feature
   <222> (1572)..(1572)
   <223> n is a, c, g, or t
<220>
   <221> misc_feature
   <222> (1581)..(1581)
   <223> n is a, c, g, or t
<220>
   <221> misc_feature
   <222> (1587)..(1587)
   <223> n is a, c, g, or t
<220>
   <221> misc_feature
   <222> (1590)..(1590)
   <223> n is a, c, g, or t
<220>
   <221> misc_feature
   <222> (1593)..(1593)
   <223> n is a, c, g, or t
<220>
   <221> misc_feature
   <222> (1596)..(1596)
   <223> n is a, c, g, or t
<220>
   <221> misc_feature
   <222> (1599)..(1599)
   <223> n is a, c, g, or t
<220>
   <221> misc_feature
   <222> (1605)..(1605)
   <223> n is a, c, g, or t
<220>
   <221> misc_feature
   <222> (1623)..(1623)
   <223> n is a, c, g, or t
<220>
   <221> misc_feature
   <222> (1629)..(1629)
   <223> n is a, c, g, or t
<220>
   <221> misc_feature
   <222> (1635)..(1635)
   <223> n is a, c, g, or t
<220>
   <221> misc_feature
   <222> (1641)..(1641)
   <223> n is a, c, g, or t
<220>
   <221> misc_feature
   <222> (1644)..(1644)
   <223> n is a, c, g, or t
<220>
   <221> misc_feature
   <222> (1662)..(1662)
   <223> n is a, c, g, or t
<220>
   <221> misc_feature
   <222> (1668)..(1668)
   <223> n is a, c, g, or t
<220>
   <221> misc_feature
   <222> (1671)..(1671)
   <223> n is a, c, g, or t
<220>
   <221> misc_feature
   <222> (1674)..(1674)
   <223> n is a, c, g, or t
<220>
   <221> misc_feature
   <222> (1677)..(1677)
   <223> n is a, c, g, or t
<220>
   <221> misc_feature
   <222> (1680)..(1680)
   <223> n is a, c, g, or t
<220>
   <221> misc_feature
   <222> (1686)..(1686)
   <223> n is a, c, g, or t
<220>
   <221> misc_feature
   <222> (1692)..(1692)
   <223> n is a, c, g, or t
<220>
   <221> misc_feature
   <222> (1701)..(1701)
   <223> n is a, c, g, or t
<220>
   <221> misc_feature
   <222> (1704)..(1704)
   <223> n is a, c, g, or t
<220>
   <221> misc_feature
   <222> (1722)..(1722)
   <223> n is a, c, g, or t
<220>
   <221> misc_feature
   <222> (1728)..(1728)
   <223> n is a, c, g, or t
<220>
   <221> misc_feature
   <222> (1737)..(1737)
   <223> n is a, c, g, or t
<220>
   <221> misc_feature
   <222> (1749)..(1749)
   <223> n is a, c, g, or t
<220>
   <221> misc_feature
   <222> (1755)..(1755)
   <223> n is a, c, g, or t
<220>
   <221> misc_feature
   <222> (1758)..(1758)
   <223> n is a, c, g, or t
<220>
   <221> misc_feature
   <222> (1764)..(1764)
   <223> n is a, c, g, or t
<220>
   <221> misc_feature
   <222> (1767)..(1767)
   <223> n is a, c, g, or t
<220>
   <221> misc_feature
   <222> (1770)..(1770)
   <223> n is a, c, g, or t
<220>
   <221> misc_feature
   <222> (1776)..(1776)
   <223> n is a, c, g, or t
<220>
   <221> misc_feature
   <222> (1785)..(1785)
   <223> n is a, c, g, or t
<220>
   <221> misc_feature
   <222> (1794)..(1794)
   <223> n is a, c, g, or t
<220>
   <221> misc_feature
   <222> (1803)..(1803)
   <223> n is a, c, g, or t
<220>
   <221> misc_feature
   <222> (1806)..(1806)
   <223> n is a, c, g, or t
<220>
   <221> misc_feature
   <222> (1821)..(1821)
   <223> n is a, c, g, or t
<220>
   <221> misc_feature
   <222> (1824)..(1824)
   <223> n is a, c, g, or t
<220>
   <221> misc_feature
   <222> (1827)..(1827)
   <223> n is a, c, g, or t
<220>
   <221> misc_feature
   <222> (1830)..(1830)
   <223> n is a, c, g, or t
<220>
   <221> misc_feature
   <222> (1836)..(1836)
   <223> n is a, c, g, or t
<220>
   <221> misc_feature
   <222> (1839)..(1839)
   <223> n is a, c, g, or t
<220>
   <221> misc_feature
   <222> (1848)..(1848)
   <223> n is a, c, g, or t
<400> 3
   atgggnwsng gnytnttyaa rccnmgngtn cayccngayy tnmgngaygt nttywsnaar      60
   atgwsnttyt tygayaarat hggnttyytn ttyathcayg cnttygayaa rmgnaayytn     120
   tggcayaarg tnccngtncc nathggnytn ytntayytna ayacnmgnmg nacnytnytn     180
   garaartaya ayytnytngc ngtnggnmgn wsnwsncayg gngcnytntt ygayccnaar     240
   garttyytnt aymgnacnga rgayggnaar tayaaygayc cncayaaygc ngargcnggn     300
   wsncaraaya cnttyttygg nmgnaayatg garccngtng aycarcarga ygarytnatg     360
   wsnccngayc cnttygtngt ngcnacnaar ytnytngcnm gnmgngarta yaargayacn     420
   ggnaarcart tyaayathyt ngcngcngcn tggathcart tyatggtnca ygaytggatg     480
   gaycayatgg argayacngg ncarathggn athacngcnc cnaargargt ngcnaaygar     540
   tgyccnytna arwsnttyaa rttycayccn acnaargary tnccnacnaa ywsngayggn     600
   athaarathg gncaytayaa yathmgnacn gcntggtggg ayggnwsngc ngtntayggn     660
   aayaaygarg armgngcnga raarytnmgn acntaygtng ayggnaaryt ngtnathggn     720
   gaygayggny tnytnytnca yaargaraay ggngtngcny tnwsnggnga yathmgnaay     780
   wsntgggcng gngtnwsnat hytncargcn ytnttygtna argarcayaa ygcngtntgy     840
   gaygcnatha argargarca yccnaayytn wsngaygarg arytntaymg ntaygcnaar     900
   ytngtnacnw sngcngtnat hgcnaargtn cayacnathg aytggacngt ngarytnytn     960
   aaracnaara cnatgmgngc ngcnatgmgn gcnaaytggt ayggnytnyt nggnaaraar    1020
   athaargaya cnttyggnca yathggnggn ccnathytng gnggnytngt nggnytnaar    1080
   aarccnaaya aycayggngt nccntaywsn ytnacngarg arttyacnws ngtntaymgn    1140
   atgcaywsny tnathccnws nacnytnaar ytnmgngayc cnacnggnca rccngaygcn    1200
   aayaaywsnc cnccntgyyt ngargayath gayathggng aratgathgg nytnaarggn    1260
   gargarcary tnwsnaarat hggnttygar aarcargcny tnwsnatggg ntaycargcn    1320
   tgyggngcny tngarytntg gaaytayccn wsnttyttym gnaayytnat hccncaraay    1380
   ytngayggna cnaaymgnws ngaymgnath gayytngcng cnytngargt ntaymgngay    1440
   mgngarmgnw sngtnccnmg ntayaaygar ttymgnmgnm gnytnttyyt nathccnath 1500
   aarwsntggg argayytnac nwsngayaar gaygcnathg aracnathmg ngcnathtay 1560
   ggngaygayg tngaraaryt ngayytnytn gtnggnytna tggcngaraa raarathaar 1620
   ggnttygcna thwsngarac ngcnttyaay athttyathy tnatggcnws nmgnmgnytn 1680
   gargcngaym gnttyttyac nwsnaaytty aaygargara cntayacnaa raarggnatg 1740
   cartgggtna aracnacnga rggnytnmgn gaygtnatha aymgncayta yccngarath 1800
   acngcnaart ggatgaarws nwsnwsngcn ttywsngtnt gggaygcnga ytay 1854
<210> 4
   <211> 1854
   <212> DNA
   <213> Oryza sativa
<400> 4
   atgggcagcg gcctgtttaa accgcgcgtg catccggatc tgcgcgatgt gtttagcaaa      60
   atgagctttt ttgataaaat tggctttctg tttattcatg cgtttgataa acgcaacctg     120
   tggcataaag tgccggtgcc gattggcctg ctgtatctga acacccgccg caccctgctg     180
   gaaaaatata acctgctggc ggtgggccgc agcagccatg gcgcgctgtt tgatccgaaa     240
   gaatttctgt atcgcaccga agatggcaaa tataacgatc cgcataacgc ggaagcgggc     300
   agccagaaca ccttttttgg ccgcaacatg gaaccggtgg atcagcagga tgaactgatg     360
   agcccggatc cgtttgtggt ggcgaccaaa ctgctggcgc gccgcgaata taaagatacc     420
   ggcaaacagt ttaacattct ggcggcggcg tggattcagt ttatggtgca tgattggatg     480
   gatcatatgg aagataccgg ccagattggc attaccgcgc cgaaagaagt ggcgaacgaa     540
   tgcccgctga aaagctttaa atttcatccg accaaagaac tgccgaccaa cagcgatggc     600
   attaaaattg gccattataa cattcgcacc gcgtggtggg atggcagcgc ggtgtatggc     660
   aacaacgaag aacgcgcgga aaaactgcgc acctatgtgg atggcaaact ggtgattggc     720
   gatgatggcc tgctgctgca taaagaaaac ggcgtggcgc tgagcggcga tattcgcaac     780
   agctgggcgg gcgtgagcat tctgcaggcg ctgtttgtga aagaacataa cgcggtgtgc     840
   gatgcgatta aagaagaaca tccgaacctg agcgatgaag aactgtatcg ctatgcgaaa     900
   ctggtgacca gcgcggtgat tgcgaaagtg cataccattg attggaccgt ggaactgctg     960
   aaaaccaaaa ccatgcgcgc ggcgatgcgc gcgaactggt atggcctgct gggcaaaaaa    1020
   attaaagata cctttggcca tattggcggc ccgattctgg gcggcctggt gggcctgaaa    1080
   aaaccgaaca accatggcgt gccgtatagc ctgaccgaag aatttaccag cgtgtatcgc    1140
   atgcatagcc tgattccgag caccctgaaa ctgcgcgatc cgaccggcca gccggatgcg    1200
   aacaacagcc cgccgtgcct ggaagatatt gatattggcg aaatgattgg cctgaaaggc    1260
   gaagaacagc tgagcaaaat tggctttgaa aaacaggcgc tgagcatggg ctatcaggcg    1320
   tgcggcgcgc tggaactgtg gaactatccg agcttttttc gcaacctgat tccgcagaac    1380
   ctggatggca ccaaccgcag cgatcgcatt gatctggcgg cgctggaagt gtatcgcgat    1440
   cgcgaacgca gcgtgccgcg ctataacgaa tttcgccgcc gcctgtttct gattccgatt    1500
   aaaagctggg aagatctgac cagcgataaa gatgcgattg aaaccattcg cgcgatttat    1560
   ggcgatgatg tggaaaaact ggatctgctg gtgggcctga tggcggaaaa aaaaattaaa    1620
   ggctttgcga ttagcgaaac cgcgtttaac atttttattc tgatggcgag ccgccgcctg    1680
   gaagcggatc gcttttttac cagcaacttt aacgaagaaa cctataccaa aaaaggcatg    1740
   cagtgggtga aaaccaccga aggcctgcgc gatgtgatta accgccatta tccggaaatt    1800
   accgcgaaat ggatgaaaag cagcagcgcg tttagcgtgt gggatgcgga ttat          1854

## Beschreibung

Die vorliegende Erfindung betrifft das Gebiet der Herstellung aliphatischer Aldehyde mit 5 bis 31 Kohlenstoffatome, insbesondere durch mikrobielle Umsetzung entsprechender aliphatischer Fettsäuren mit 6 bis 32 Kohlenstoffatomen. Im Zusammenhang mit der Erfindung werden auch Enzyme zum Katalysieren einer solchen Umsetzungsreaktion sowie dafür codierende Nukleinsäuren beschrieben.

Die enzymatische Herstellung von Aldehyden ist dem Grunde nach bereits bekannt. So beschreiben Muller et al. in US 5464761 unter anderem die Herstellung aliphatischer Aldehyde aus Linolsäure. Ebenfalls bekannt ist die Herstellung von C6-C10 Aldehyden aus ungesättigten Triacylglyceriden (EP 1244364 B1) mittels Lipoxygenasen, dabei entstehen jedoch Aldehydgemische.

Binder beschreibt in "Enzymatic method of making aldehydes from fatty acids". 2009, Archer-Daniels-Midland Company, die Umsetzung von pflanzlichen und tierischen Fette zu den korrespondierenden Aldehyden durch eine Fettsäure-Reduktase. Diese Umsetzung ist jedoch abhängig von Cofaktoren zur Aktivierung der Fettsäure durch die Bildung von Acyl-AMP, sie lässt sich daher schwerlich im Produktionsmaßstab statt im Labormaßstab durchführen.

Saffert et al. beschreiben in "A dual function alpha-dioxygenase-peroxidase and NAD(+) oxidoreductase active enzyme from germinating pea rationalizing alpha-oxidation of fatty acids in plants", Plant Physiol, 2000, 1545-52, die Umsetzung von Hexadecansäure zu 2-Hydroperoxyhexadecansäure und 2-Hydroxyhexadecansäure durch eine gereinigte alpha-Dioxygenase, dabei wurden als Nebenprodukte Pentadecansäure und, geringfügig, Pentadecanal beobachtet.

Ferner bekannt ist die Umsetzung von Linolensäure, Linolsäure und Ölsäure zu den entsprechenden Hydroxysäuren sowie den entsprechend um 1 Kohlenstoffatom verkürzten Fettsäuren und Aldehyden (Hamberg et al., "alpha-oxidation of fatty acids in higher plants". J Biol Chem 1999, 24503-24513), dabei erfolgte die Umsetzung mit einem Protein-Rohextrakt aus Insektenzellen, die eine pathogen-induzierbare Oxygenase ("PIOX") heterolog exprimierten.

Nachteilig an herkömmlichen Verfahren ist jedoch, dass darin aus Zellen isolierte und ggf. aufgereinigte Enzyme verwendet werden müssen. Dies macht die Verfahren aufwendig, indem sie zusätzliche Schritte für das Isolieren und ggf. für das Aufreinigen des jeweiligen Enzyms erfordern, und weitere Maßnahmen erforderlich sind, das isolierte und/oder aufgereinigte Enzym zu stabilisieren. Bei einem großtechnischen Verfahren müsste das isolierte und/oder aufgereinigte Enzym zusätzlich immobilisiert werden, oder es müssten Einrichtungen vorgesehen werden, um nach Durchführen der Umsetzung das Enzym aus dem Reaktionsansatz rückzugewinnen. Nachteilig ist ferner, dass wie oben beschrieben in einigen Fällen Co-Faktoren zur Bereitstellung von Reduktionsäquivalenten (NAD(P)H) und/oder zur Bereitstellung von Energie (ATP) benötigt werden, so dass die Anforderungen für das Einhalten optimaler oder zumindest großtechnisch brauchbarer Reaktionsbedingungen zusätzlich verkompliziert werden.

Die US 7491854 B2 zeigt ein Ganzzell-Biotransformationsverfahren zum Herstellen von Aldehyden aus Fettsäure. Dabei wird Oleinsäure durch E. coli-Zellen, die heterolog eine Nocardia-Karbonsäurereduktase exprimieren, während eines Wachstumszeitraums von 24 Stunden unter anderem zum entsprechenden Aldehyd umgesetzt. Für eine entsprechende In vitro-Umsetzung wurden als Co-Faktoren ATP und NADPH benötigt. Die Umsetzung in einem Ganzzell-Biotransformationsverfahren kann deshalb ebenfalls nur mit wachsenden Zellen durchgeführt werden, die entsprechend ATP und NADPH bilden können. Dies hat auch zur Folge, dass ein Teil der eingesetzten Fettsäure von den wachsenden Zellen beispielsweise im Rahmen der Beta-Oxidation verstoffwechselt wird und somit nicht für die Herstellung eines Aldehyds zur Verfügung steht.Nachteilig ist ferner, dass bei einer Umsetzung mit einer Karbonsäurereduktase wie in der US 7491854 B2 beschrieben die Kettenlänge der Fettsäure auch die Kettenlänge des Aldehyds ist. Viele wirtschaftlich interessante Aldehyde besitzen eine ungerade Anzahl an Kohlenstoffatomen; Fettsäuren mit einer ungeraden Anzahl an Kohlenstoffatomen sind jedoch im Regelfalle deutlich teurer als nächstlängere bzw. nächstkürzere geradzahlige Fettsäuren.

Es war deshalb die Aufgabe der vorliegenden Erfindung, den oben beschriebenen Nachteilen abzuhelfen und ein Verfahren zum Herstellen von Aldehyden aus Fettsäuren anzugeben. Ferner sollen Nucleinsäuren, Enzyme und Mikroorganismen zum Durchführen eines solchen Verfahrens bereitgestellt werden.

Erfindungsgemäß wird deshalb ein Verfahren zum Herstellen eines Aldeyhds mit 5 bis 31 Kohlenstoffatomen bereitgestellt, umfassend die Schritte:
a) Bereitstellen von Mikroorganismenzellen enthaltend eine alpha-Dioxygenase,
b) Beaufschlagen der Mikroorganismenzellen mit einem Umsetzungsmedium enthaltend eine Fettsäure mit 6 bis 32 Kohlenstoffatomen, und
c) Umsetzen der Fettsäure zum Aldehyd durch die Dioxygenase.

Überraschenderweise hat sich herausgestellt, dass auch bei Verwendung intakter Mikroorganismenzellen wie beispielsweise E. coli in wirtschaftlich erheblichem Maße Fettsäuren in die Mikroorganismenzellen transportiert werden, ohne unmittelbar der Beta-Oxidation zugeführt zu werden. Bisher war erwartet worden, dass Fettsäuren hauptsächlich durch eine Acyl-CoA-Synthetase durch die innere Zellmembran transportiert werden. Dieses Enzym setzt jedoch die zu transportierende Fettsäure zum entsprechenden CoA-Thioester um, so dass dieser Fettsäuretransport für die Mikroorganismenzelle energieaufwendig ist; zum anderen werden CoA-Fettsäurethioester unmittelbar der Beta-Oxidation zugeführt und in wachsenden Zellen entsprechend abgebaut, so dass kein brauchbares Aldehyd gebildet wird. Überraschenderweise hat sich nun herausgestellt, dass beispielsweise in E. coli ein zweiter Fettsäuretransportweg neben dem durch die Acyl-CoA-Synthetase vermittelten Transportweg zur Verfügung stehen muss, mit dem Fettsäuren auch von beispielsweise ruhenden Zellen aufgenommen und der Dioxygenase zugänglich gemacht werden können.

Es ist deshalb nunmehr auch in wirtschaftlich relevanten Verfahren möglich, also jenseits des Labormaßstabs, durch eine Biotransformation Aldehyde aus entsprechenden Fettsäuren herzustellen, ohne dabei auf ein energiereiches CoA-Coenyzm zurückgreifen zu müssen und der jeweiligen Mikroorganismenzelle entsprechend Energie zuzuführen, beispielsweise durch Beta-oxidativen Abbau der Fettsäure.

Das erfindungsgemäße Verfahren vermeidet so auch die Notwendigkeit eines Zellaufschlusses und einer Aufreinigung der Dioxygenase. Nachteilig an einem Zellaufschluss wäre insbesondere, dass dabei ein komplexes Medium entsteht, in dem verschiedenste kaum steuerbare Reaktionen ablaufen und aus dem die Produktgewinnung unnötig kompliziert wird. Bei einer erfindungsgemäßen Ganzzell-Biotransformation lassen sich hingegen die verwendeten Mikroorganismen ohne Weiteres vom umgebenden Medium abtrennen. Zudem können sie nach Abtrennung vom umgebenden Medium einem weiteren Biotransformations-Ansatz zugeführt werden, so dass die Rückgewinnung der Dioxygenase im Vergleich mit einem entsprechenden Verfahren unter Verwendung eines isolierten und/oder aufgereinigten Enzyms deutlich vereinfacht und mit geringeren Kosten versehen ist. Darüber hinaus wird das Produkt, also das entsprechende Aldehyd, bei geeigneter Verfahrensführuhg in das Medium abgegeben, sodass das Produkt für eine Aufreinigung leichter zugänglich ist.

Im Sinne der vorliegenden Erfindung ist eine Mikroorganismenzelle eine Zelle mit intakter Zellmembran; die erfindungsgemäß eingesetzten Mikroorganismenzellen können also mit üblichen Kultivierungsverfahren wachsen gelassen werden und in dieser Form, ohne zusätzliches Perforieren der Zellmembran, eingesetzt werden.

Eine alpha-Dioxygenase im Sinne der vorliegenden Erfindung ist ein Enzym, das die Umsetzung einer Karboxcylgruppe mit O₂ katalysiert. Diese katalysieren die Umsetzung einer Karbonsäure gemäß nachfolgendem Schema: 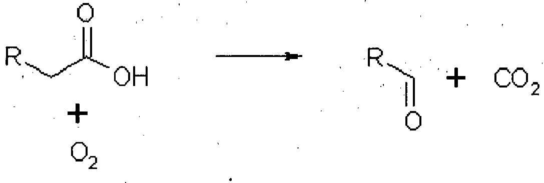 wobei R für den Rest der Fettsäure steht. Alpha-Dioxygenasen katalysieren daher die Umsetzung einer Fettsäure zu einem entsprechenden um 1 C-Atom verkürzten Aldehyd (vgl. Saffert et al., a.a.O.).

Die in einem erfindungsgemäßen Verfahren umgesetzten Fettsäuren sind vorzugsweise aliphatische Fettsäuren. Sie können verzweigt oder unverzweigt sein. Ferner können sie gesättigt oder ungesättigt sein. Sie können ferner ungesättigt oder gesättigt sein und dabei 1-5 Substituenten tragen, wobei die Substituenten jeweils unabhängig ausgewählt sind aus Hydroxy, C1-C10-Alkyl, C1-C10-Alkoxy, C6-C10-Aryl, Phenyl-C1-C5-Alkyl und Phenyl-C1-C5-Alkenyl, wobei das alpha-C-Atom der Fettsäure keinen solchen Substituenten trägt.

Vorzugsweise ist die Fettsäure zum Bilden des Aldehyds ausgewählt aus:

| Fettsäure | zum Bilden von |
|---|---|
| n-Heptansäure | n-Hexanal |
| n-Nonansäure | n-Octanal |
| n-Decansäure | n-Nonanal |
| n-Undecansäure | n-Decanal |
| n-Dodecansäure | n-Undecanal |
| n-Tridecansäure | n-Dodecanal |
| n-Tetradecansäure | n-Tridecanal |
| n-Hexadecansäure | n-Pentadecanal |
| 3-Methyl-Undecansäure | 2-Methyldecanal |
| 3-Methyl-Dodecansäure | 2-Methylundecanal |
| trans-3-Heptensäure | trans-2-Hexenal |
| cis-5-Octansäure | cis-4-Heptenal |
| 3,7-Dimethyl-6-Octensäure | 2,6-Dimethyl-5-Hepten-1-al |
| 11-Dodecensäure | 10-Undecenal |
| 3,7,11-Trimethyl-6,10-Dodecadiensäure | 2,6,10-Trimethyl-5,9-undecadienal |
| 4,8-Dimethyl-7-nonen-1-säure | Citronellal |
| n-Heptadecansäure | n-Hexadecanal |
| n-Octadecansäure | n-Heptadecanal |
| n-Nonadecansäure | n-Octadecanal |

Für die Zwecke der vorliegenden Erfindung sind insbesondere folgende Fettsäuren bevorzugt:

| Fettsäure | Zugehöriger Aldehyd |
|---|---|
| n-Hexadecansäure (Palmitinsäure) | n-Pentadecanal |
| n-Tetradecansäure (Myristinsäure) | n-Tridecanal |
| n-Dodecansäure (Laurinsäure) | n-Undecanal |
| n-Decansäure (Caprinsäure) | n-Nonanal |

Die Verwendung einer alpha-Dioxygenase ermöglicht also auf vorteilhaft einfache Weise die Herstellung eines Aldehyds mit einer bezogen auf die eingesetzte Fettsäure um 1 Kohlenstoffatom verringerten Kettenlänge. Damit ist dieses Verfahren besonders geeignet zum Herstellen von Aldehyden mit einem ungeradzahligen Kohlenstoffrückgrat. Dementsprechend lassen sich aus preiswert erhältlichen Fettsäuren mit gerader Anzahl an Kohlenstoffatomen im Fettsäurerückgrat die zugehörigen Aldehyde mit ungerader Anzahl an Kohlenstoffatomen im Kettenrückgrat bilden.

Das erfindungsgemäße Verfahren kann auch so durchgeführt werden, dass mehrere verschiedene Fettsäuren gleichzeitig zur Umsetzung durch die Dioxygenase vorgelegt werden.

Vorzugsweise wird ein erfindungsgemäßes Verfahren, insbesondere eines mit einer alpha-Dioxygenase, mit solchen Mikroorganismenzellen durchgeführt, bei denen zumindest in Schritt c) und vorzugsweise auch in Schritt b) die Beta-Oxidation im Vergleich zu einem Wildtyp-Stamm herabgesetzt ist. Dies kann beispielsweise durch Verabreichen eines Beta-Oxidations-Inhibitors an die Mikroorganismen vor Schritt c) oder gleichzeitig mit Schritt b) erfolgen.

Erfindungsgemäß bevorzugt ist jedoch ein solches Verfahren, bei dem nicht-wachsende, ruhende Mikroorganismenzellen eingesetzt werden. Dementsprechend bevorzugt ist es, wenn das Medium in Schritt b) einen Maximalgehalt von 0,1 ppm an Stickstoff-Verbindungen besitzt, vorzugsweise beträgt der Gehalt des Mediums an stickstoffhaltigen Verbindungen höchstens 0,01 ppm und besonders bevorzugt höchstens 0,001 ppm. Dementsprechend bevorzugt ist ein erfindungsgemäßes Verfahren, bei dem das Medium in Schritt b) keine für die Mikroorganismenzellen verwertbare Stickstoffquelle besitzt.

Ein bevorzugtes Umsetzungsmedium zum Durchführen eines erfindungsgemäßen Verfahrens besteht deshalb aus Wasser, einer oder mehreren umzusetzenden Fettsäure(n), optional einem stickstofffreien Lösungsvermittler für den oder die Fettsäure(n), optional einem stickstofffreien pH-Puffer und optional einem stickstofffreien Nährsubstrat für die Mikroorganismenzellen. Als pH-Puffer bevorzugt sind Natrium- und/oder Kaliumphosphatpuffer; als Nährsubstrat bevorzugt ist Glukose oder eine andere von den Mikroorganismenzellen verstoffwechselbare Substanz, soweit diese Substanz verglichen mit der oder den umzusetzenden Fettsäure(n) bevorzugt verstoffwechselt wird. Dabei bedeutet "bevorzugt verstoffwechseln", dass durch Zusatz der Substanz zum Umsetzungsmedium bei ansonsten unveränderten Umsetzungsbedingungen der Quotient aus den Konzentrationen von
a) gewünschtem Aldehyd zu umzusetzender Fettsäure, bzw.
b) wenn mehrere Fettsäuren umzusetzen sind, von allen gewünschten Aldehyden zu allen gewünschten Fettsäuren,
zumindest gleich bleibt.

Ein erfindungsgemäß bevorzugter Lösungsvermittler für Fettsäuren ist Dimethylsulfoxid (DMSO) und Triton X 100.

Für die Zwecke der vorliegenden Erfindung bevorzugt ist insbesondere eine alpha-Dioxygenase mit einer Peroxidase-Aktivität von höchstens 0,4 nkat/mg gemessen mit einem chromatofokussierten, aufgereinigten Enzym mit 2-Hydroperoxypalmitinsäure und spektrofotometrischer Bestimmung des Oxidationsprodukts von Guaiacol bei 470 nm. Dieses Bestimmungsverfahren ist dem Fachmann beispielsweise bekannt aus Maehly, "Plant peroxidases", Methods Enzymol (1955), 801 bis 813. Besonders bevorzugt beträgt die Peroxidase-Aktivität höchstens 0,3 nkat/mg; ganz besonders bevorzugt besitzt die Dioxygenase keine derart nachweisbare Peroxidase-Aktivität.

In einem erfindungsgemäßen Verfahren besitzt die Dioxygenase vorzugsweise eine Aminosäuresequenz-Ähnlichkeit "similarity" zur Aminosäuresequenz SEQ ID No. 1 von zumindest 80%, vorzugsweise zumindest 88%, mehr bevorzugt zumindest 93% und besonders bevorzugt zumindest 98%. Im Sinne der vorliegenden Erfindung werden Aminosäuresequenz-Ähnlichkeiten bestimmt anhand des Waterman-Smith-Algorhythmus mit einer Lückeneröffnungs-Strafe ("gap open penalty") von 10, einer Lückenvergrößerungs-Strafe ("gap extension penalty") von 0,5 und der BLOSUM62-Matrix. Der Waterman-Smith-Algorithmus ist beschrieben in Smith, T. F. and Waterman, M. S., "Identification of common molecular subsequences", Journal of Molecular Biology (1981), 147:195-197 und beispielsweise online implementiert über die entsprechende Werkzeugseite des EMBL, derzeit "EMBOSS::water" erhältlich per www.ebi.ac.uk/tools/emboss/align/. Die diesen Randbedingungen genügenden Dioxygenasen besitzen für die Zwecke des erfindungsgemäßen Verfahrens eine gute Aktivität für die Umsetzung von Fettsäuren zu um 1 Kohlenstoffatom verkürzten entsprechenden Aldehyden. Sie sind in einer Vielzahl von Mikroorganismen für ein Ganzzell-Biotransformationsverfahren der erfindungsgemäßen Art gut exprimierbar. Entsprechende Dioxygenasen sind bereits bekannt. Ihre Verwendbarkeit zum Herstellen von Aldehyden in einem Ganzzell-Biotransformationsverfahren gemäß der vorliegenden Erfindung war jedoch unbekannt.

M. Hamberg et al. beschreiben in einem Übersichtsartikel "α-Dioxygenases" in Biochem. Biophys. Res. Comm.; 2005, Seiten 169-174, die Familie der alpha-Dioxygenasen, die die Oxidation von Cn-Fettsäuren in Cn-1-Aldehyde katalysieren.

In Prostagl. & Other Lipid Mediators.; 2002, Seiten 363-374 geben Hamberg et al. einen Überblick über die Biochemie von alpha-Dioxygenasen und Lipoxygenasen aus verschiedenen Pflanzen.

Besonders bevorzugt ist ein erfindungsgemäßes Verfahren, bei dem die Dioxygenase eine alpha-Dioxygenase aus einer Pflanze der Gattung Oryza ist, vorzugsweise aus Reis (Oryza sativa). Derartige alpha-Dioxygenasen sind beispielsweise bekannt aus Koeduka et al., "Catalytic properties of rice alpha-oxigenase", J. Biol. Chem. 2002, 22648 bis 22655. Derartige alpha-Dioxygenasen insbesondere aus Reis sind in E. coli leicht und mit guter Stabilität und Aktivität heterolog exprimierbar. Besonders vorteilhaft ist, dass sie über keine nennenswerte Peroxidase-Aktivität verfügen. Erfindungsgemäß bevorzugt ist dementsprechend eine Alpha-Dioxygenase mit einer Aminosäuresequenz gemäß SEQ ID No. 1.

Die Aminosäure- bzw. Gensequenz der alpha-Dioxygenase aus *Oryza sativa* ist in den Datenbanken NCBI NP_001066718; Seiten 1-4 oder GenBank AAF64042.2 (Version 2), 2007 veröffentlicht.

Ferner besonders bevorzugt ist ein erfindungsgemäßes Verfahren, bei dem die Mikroorganismenzellen ausgewählt sind aus
- der Klasse der Gamma-Proteobakterien, vorzugsweise der Familie der Enterobacteriaceae und besonders bevorzugt aus den Gattungen Escherichia (darin wiederum besonders bevorzugt Escherichia coli),
- der Klasse der Bacilli, darin bevorzugt der Gattung Bacillus oder der Ordnung der Lactobacillales, und
- der Klasse der Saccharomyceten, dabei besonders bevorzugt der Familie der Saccharomycetaceae und der Dipodascaceae, und besonders bevorzugt der Gattung Saccharomyces und Yarrowia.

Besonders bevorzugt sind die Mikroorganismenzellen in einem erfindungsgemäßen Verfahren solche des Genus Escherichia, insbesondere Escherichia coli ist besonders bevorzugt. Derartige Mikroorganismen lassen sich leicht und sicher handhaben; der Umgang mit ihnen ist auch großtechnisch gut etabliert, und mit ihnen lassen sich gute Ausbeuten erzielen.

Dementsprechend bevorzugt ist ein erfindungsgemäßes Verfahren zum Herstellen eines Aldehyds mit 5 bis 31 Kohlenstoffatomen, umfassend die Schritte:
a) Bereitstellen von E. coli-Zellen enthaltend eine alpha-Dioxygenase mit einer Ähnlichkeit wie oben beschrieben von zumindest 98% zu einer Aminosäuresequenz gemäß SEQ ID No. 1,
b) Beaufschlagen der Mikroorganismen mit einem Umsetzungsmedium enthaltend eine Fettsäure mit 6 bis 32 Kohlenstoffatomen, wobei das Medium einen Gehalt an Stickstoff-Verbindungen von weniger als 0,1 ppm besitzt, und optional Glucose enthält, und
c) Umsetzen der Fettsäure zum entsprechenden Aldehyd mit einem um 1 Kohlenstoffatom verringerten Kohlenstoffrückgrat.

Der pH-Wert eines Umsetzungsmediums wird erfindungsgemäß - vorzugsweise mit Kaliumphosphat - auf einen Wert von bevorzugt 6,5 bis 9 eingestellt, mehr bevorzugt 7 bis 8 und besonders bevorzugt 7,3 bis 7,7. Diese pH-Werte lassen insbesondere bei Verwendung einer alpha-Dioxygenase aus Reis wie oben beschrieben in stickstofflimitierten E. coli-Zellen gute Umsätze (mmol Aldehyd pro mmol Fettsäure, Zeit und Kubikmeter Umsetzungsmedium) erzielen.

Ebenfalls bevorzugt ist es, das Umsetzen in Schritt c) bei einer Temperatur von 25 bis 39°C, vorzugsweise von 28 bis 32°C und besonders bevorzugt von 29 bis 31°C durchzuführen. Diese Temperaturen sind insbesondere an übliche E. coli-Produktionsstämme zum Erreichen eines wie vorstehende beschriebenen guten Umsatzes mit einem erfindungsgemäßen Verfahren angepasst.

Im Zusammenhang mit der vorliegenden Erfindung wird ferner ein Produktionsstamm angegeben, der eine Dioxygenase wie oben beschrieben heterolog exprimiert. Der Produktionsstamm ist dabei vorzugsweise ausgewählt aus
- der Klasse der Gamma-Proteobakterien, vorzugsweise der Familie der Enterobacteriaceae und besonders bevorzugt aus den Gattungen Escherichia (darin wiederum besonders bevorzugt Escherichia coli),
- der Klasse der Bacilli, darin bevorzugt der Gattung Bacillus oder der Ordnung der Lactobacillales, und
- der Klasse der Saccharomyceten, dabei besonders bevorzugt der Familie der Saccharomycetaceae und der Dipodascaceae, und besonders bevorzugt der Gattung Saccharomycesund Yarrowia.

Die vom Produktionsstamm, vorzugsweise einem Mikroorganismus der Art Escherichia coli, exprimierte Dioxygenase ist vorzugsweise eine alpha-Dioxygenase mit einer wie oben definierten Ähnlichkeit ("similarity") zur Aminosäuresequenz SEQ ID No. 1 von zumindest 80%, vorzugsweise zumindest 88% und besonders bevorzugt zumindest 93%. Mit den erfindungsgemäßen Produktionsstämmen lassen sich die oben beschriebenen Vorteile des erfindungsgemäßen Verfahrens verwirklichen.

In Zusammenhang mit der vorliegenden Erfindung wird ferner eine Nukleinsäure angegeben zur Transformation eines Mikroorganismenstamms zum Erhalten eines erfindungsgemäßen Produktionsstammes, wobei die Nukleinsäure ein Gen umfasst, das für eine Dioxygenase kodiert mit einer Aminosäuresequenz-Ähnlichkeit ("similarity") zur Aminosäuresequenz SEQ ID No. 1 von zumindest 80%, vorzugsweise zumindest 88%, mehr bevorzugt zumindest 93% und besonders bevorzugt zumindest 98%, jeweils gemessen wie oben beschrieben, und wobei das Gen heterolog zur übrigen Nukleinsäure ist. Die Nukleinsäure ist vorzugsweise ein DNA-Vektor, insbesondere bevorzugt ein Rekombinations-Vektor zum stabilen Einbau des Gens in das Genom eines Mikroorganismus durch Rekombination, oder ein episomaler Vektor.

Das Gen der Nukleinsäure ist vorzugsweise codonoptimiert für den zugeordneten, zu transformierenden Mikroorganismus. Besonders bevorzugt besitzt eine erfindungsgemäße Nukleinsäure und ein erfindungsgemäßer Produktionsstamm daher ein Gen mit einer Sequenz gemäß SEQ ID No. 3 und besonders bevorzugt mit einer Sequenz gemäß SEQ ID No. 4. Mit derartigen Genen können insbesondere in Escherichia coli-Produktionsstämmen Dioxygenasen mit einer Aminosäuresequenz gemäß SEQ ID No. 1 exprimiert und die mit der Verwendung dieser Dioxygenase verbundenen Vorteile verwirklicht werden.

Das Gen zur erfindungsgemäßen Expression der alpha-Dioxygenase ist vorzugsweise unter der Kontrolle eines induzierbaren Promotors exprimierbar. Auf diese Weise kann erreicht werden, dass die Dioxgenaseexpression erst kurz vor oder während der Umsetzungsreaktion (Schritt c) erfolgt. Dies vermeidet ein langsameres Zellwachstum während der Anzucht der erfindungsgemäß eingesetzten Mikroorganismen. Vorzugsweise steht das Gen unter der Kontrolle eines mit dem *lac*-Repressor schaltbaren Promotors, so dass durch Verabreichen beispielsweise von Isopropyl-β-D-1-thiogalactopyranosid (IPTG) die Expression des Dioxygenase-Gens induziert werden kann.

Die Erfindung wird nachfolgend anhand der Beispiele näher erläutert, ohne dass diese den Schutzbereich der Patentansprüche beschränken sollen.

### Beispiel 1

### Herstellung von Pentadecanal aus n-Hexadecansäure

Die Anzucht einer Vorkultur des *E. coli*-Stammes BL21 (DE3) pET-28a αDOX-Reis erfolgte in 5 ml LB-Medium mit 30 µg Kanamycin pro ml Medium bei 37°C für ca. 6 h.

### LB-Medium:

5 g Trypton
10 g Hefeextrakt
5 g Natriumchlorid
ad 1l Wasser

### Erste Phase: Anzucht

Für die Hauptkultur wurde 500 ml LB-Medium mit 30 µg Kanamycin pro ml Medium in einem 2 I- Schüttelkolben mit 1% (v/v) Vorkultur angeimpft. Die Anzucht erfolgte bei 37°C bis zu einer OD₆₀₀ₙₘ von 0,6.

### Zweite Phase: Induktion und Expression

Nach Erreichen dieser OD erfolgte die Induktion mit 0,5 mM Isopropyl-β-D-thiogalactopyranosid (IPTG) und die anschließende Expression bei einer Temperatur von 22°C über Nacht (ca. 14h).

### Dritte Phase: Biotransformation

Die Zellsuspension wurde in ein steriles Zentrifugengefäß überführt und bei 8.000g und 4°C für 10 min zentrifugiert. Das überschüssige Medium wurde durch Dekantieren entfernt und das verbleibende Zellpellet zum Waschen in sterilem Umsetzungsmedium gelöst. Anschließend wurde erneut bei 8.000g und 4°C für 10 min zentrifugiert und das Umsetzungsmedium ebenfalls durch Dekantieren entfernt. Verbleibende Flüssigkeit wurde mit einer sterilen Pipette entfernt und das Zellpellet gewogen. Daraus ergab sich ca. 2 g Zellnassmasse pro 500 ml Kultur. Um eine Konzentration von 20 g Zellnassmasse pro I zu erhalten, wurde das Pellet in 100 ml sterilem Umsetzungsmedium resupendiert.

### Umsetzungsmedium

200 mM Kaliumphosphatpuffer
0,5 % (w/v) Glucose
pH 7,5

Die Biotransformation wurde in einem 300 ml-Schüttelkolben mit Schikane mit einem Volumen von 25 ml durchgeführt. Die Zugabe des Substrates Hexadecansäure erfolgte gelöst in Dimethylsulfoxid (DMSO). Dazu wurde 100 mM Hexadecansäure in DMSO gelöst und 1,25 ml dieser Lösung der Zellsuspension zugesetzt, sodass eine Endkonzentration von 5 mM im Biotransformationsansatz erreicht wurde. Vor Zugabe der Hexadecansäure wurde die Zellsupension im Schüttelkolben im Inkubator unter Schütteln auf 30°C vorgewärmt.

Die Biotransformation fand bei 30°C unter starkem Schütteln statt. Nach ca. 2-3 h wurde eine Pentadecanalkonzentration von 4 mM erreicht. Das heißt der Umsatz betrug 80%.

### Beispiel 2

### Herrstellung von Nonanal aus n-Decansäure

Anzucht der Vorkultur, sowie erste und zweite Phase wie in Beispiel 1 beschrieben.

### Dritte Phase: Biotransformation

Die Zellsuspension wurde in ein steriles Zentrifugengefäß überführt und bei 8.000g und 4°C für 10 min zentrifugiert. Das überschüssige Medium wurde durch Dekantieren entfernt und das verbleibende Zellpellet zum Waschen in sterilem Umsetzungsmedium gelöst. Anschließend wurde erneut bei 8.000g und 4°C für 10 min zentrifugiert und das Umsetzungsmedium ebenfalls durch Dekantieren entfernt. Verbleibende Flüssigkeit wurde mit einer sterilen Pipette entfernt und das Zellpellet gewogen. Daraus ergab sich ca. 2 g Zellnassmasse pro 500 ml Kultur. Um eine Konzentration von 20 g Zellnassmasse pro I zu erhalten, wurde das Pellet in 100 ml sterilem Umsetzungsmedium resupendiert.

### Umsetzungsmedium:

200 mM Kaliumphosphatpuffer
0,5 % (w/v) Glucose
pH 7,5

Die Biotransformation wurde in einem 300 ml-Schüttelkolben mit Schikane mit einem Volumen von 25 ml durchgeführt. Die Zugabe des Substrates Decansäure erfolgte gelöst in Dimethylsulfoxid (DMSO). Dazu wurde 100 mM Decansäure in DMSO gelöst und 1,25 ml dieser Lösung der Zellsuspension zugesetzt, sodass eine Endkonzentration von 5 mM im Biotransformationsansatz erreicht wurde. Vor Zugabe der Decansäure wurde die Zellsupension im Schüttelkolben im Inkubator unter Schütteln auf 37°C vorgewärmt.

Die Biotransformation fand bei 37°C unter starkem Schütteln statt. Die gebildete Menge an Nonanal wurde nicht quantitativ bestimmt. Die Bildung des Produktes wurde qualitativ mittels GC/MS gezeigt.

### Beispiel 3

### Herstellung von Tridecanal aus n-Tetradecansäure

Anzucht der Vorkultur, sowie erste und zweite Phase wie in Beispiel 1 beschrieben.

### Dritte Phase: Biotransformation

Die Zellsuspension wurde in ein steriles Zentrifugengefäß überführt und bei 8.000g und 4°C für 10 min zentrifugiert. Das überschüssige Medium wurde durch Dekantieren entfernt und das verbleibende Zellpellet zum Waschen in sterilem Umsetzungsmedium gelöst. Anschließend wurde erneut bei 8.000g und 4°C für 10 min zentrifugiert und das Umsetzungsmedium ebenfalls durch Dekantieren entfernt. Verbleibende Flüssigkeit wurde mit einer sterilen Pipette entfernt und das Zellpellet gewogen. Daraus ergab sich ca. 2 g Zellnassmasse pro 500 ml Kultur. Um eine Konzentration von 20 g Zellnassmasse pro I zu erhalten, wurde das Pellet in 100 ml sterilem Umsetzungsmedium resupendiert.

### Umsetzungsmedium:

200 mM Kaliumphosphatpuffer
0,5 % (w/v) Glucose
pH 7,5

Die Biotransformation wurde in einem 300 ml-Schüttelkolben mit Schikane mit einem Volumen von 25 ml durchgeführt. Die Zugabe des Substrates Tetradecansäure erfolgte gelöst in Dimethylsulfoxid (DMSO). Dazu wurde 100 mM Tetradecansäure in DMSO gelöst und 1,25 ml dieser Lösung der Zellsuspension zugesetzt, sodass eine Endkonzentration von 5 mM im Biotransformationsansatz erreicht wurde. Vor Zugabe der Tetradecansäure wurde die Zellsupension im Schüttelkolben im Inkubator unter Schütteln auf 37°C vorgewärmt.

Die Biotransformation fand bei 37°C unter starkem Schütteln statt. Die gebildete Menge an Tridecanal wurde nicht quantitativ bestimmt. Die Bildung des Produktes wurde qualitativ mittels GC/MS gezeigt.

### Beispiel 4

### Herrstellung von Undecanal aus n-Dodecansäure

Anzucht der Vorkultur, sowie erste und zweite Phase wie in Beispiel 1 beschrieben.

### Dritte Phase: Biotransformation

Die Zellsuspension wurde in ein steriles Zentrifugengefäß überführt und bei 8.000g und 4°C für 10 min zentrifugiert. Das überschüssige Medium wurde durch Dekantieren entfernt und das verbleibende Zellpellet zum Waschen in sterilem Umsetzungsmedium gelöst. Anschließend wurde erneut bei 8.000g und 4°C für 10 min zentrifugiert und das Umsetzungsmedium ebenfalls durch Dekantieren entfernt. Verbleibende Flüssigkeit wurde mit einer sterilen Pipette entfernt und das Zellpellet gewogen. Daraus ergab sich ca. 2 g Zellnassmasse pro 500 ml Kultur. Um eine Konzentration von 20 g Zellnassmasse pro I zu erhalten, wurde das Pellet in 100 ml sterilem Umsetzungsmedium resupendiert.

### Umsetzungsmedium:

200 mM Kaliumphosphatpuffer
0,5 % (w/v) Glucose
pH 7,5

Die Biotransformation wurde in einem 300 ml-Schüttelkolben mit Schikane mit einem Volumen von 25 ml durchgeführt. Die Zugabe des Substrates Dodecansäure erfolgte gelöst in Dimethylsulfoxid (DMSO). Dazu wurde 100 mM Dodecansäure in DMSO gelöst und 1,25 ml dieser Lösung der Zellsuspension zugesetzt, sodass eine Endkonzentration von 5 mM im Biotransformationsansatz erreicht wurde. Vor Zugabe der Dodecansäure wurde die Zellsupension im Schüttelkolben im Inkubator unter Schütteln auf 37°C vorgewärmt.

Die Biotransformation fand bei 37°C unter starkem Schütteln statt. Die gebildete Menge an Undecanal wurde nicht quantitativ bestimmt. Die Bildung des Produktes wurde qualitativ mittels GC/MS gezeigt.

### Beispiel 5

### Herrstellung von Heptanal aus n-Octansäure

Anzucht der Vorkultur, sowie erste und zweite Phase wie in Beispiel 1 beschrieben.

### Dritte Phase: Biotransformation

Die Zellsuspension wurde in ein steriles Zentrifugengefäß überführt und bei 8.000g und 4°C für 10 min zentrifugiert. Das überschüssige Medium wurde durch Dekantieren entfernt und das verbleibende Zellpellet zum Waschen in sterilem Umsetzungsmedium gelöst. Anschließend wurde erneut bei 8.000g und 4°C für 10 min zentrifugiert und das Umsetzungsmedium ebenfalls durch Dekantieren entfernt. Verbleibende Flüssigkeit wurde mit einer sterilen Pipette entfernt und das Zellpellet gewogen. Daraus ergab sich ca. 2 g Zellnassmasse pro 500 ml Kultur. Um eine Konzentration von 20 g Zellnassmasse pro I zu erhalten, wurde das Pellet in 100 ml sterilem Umsetzungsmedium resupendiert.

### Umsetzungsmedium:

200 mM Kaliumphosphatpuffer
0,5 % (w/v) Glucose
pH 7,5

Die Biotransformation wurde in einem 300 ml-Schüttelkolben mit Schikane mit einem Volumen von 25 ml durchgeführt. Die Zugabe des Substrates Octansäure erfolgte gelöst in Dimethylsulfoxid (DMSO). Dazu wurde 100 mM Octansäure in DMSO gelöst und 1,25 ml dieser Lösung der Zellsuspension zugesetzt, sodass eine Endkonzentration von 5 mM im Biotransformationsansatz erreicht wurde. Vor Zugabe der Octansäure wurde die Zellsupension im Schüttelkolben im Inkubator unter Schütteln auf 37°C vorgewärmt.

Die Biotransformation fand bei 37°C unter starkem Schütteln statt. Die gebildete Menge an Heptanal wurde nicht quantitativ bestimmt. Die Bildung des Produktes wurde qualitativ mittels GC/MS gezeigt.

### Beispiel 6

### Herstellung von Pentadecanal aus n-Hexadecansäure mit Rückgewinnung der Zellen

Die Anzucht einer Vorkultur des *E*. *coli*-Stammes BL21 (DE3) pET-28a αDOX-Reis erfolgte in 5 ml LB-Medium mit 30 µg Kanamycin pro ml Medium bei 37°C für ca. 6 h.

### LB-Medium:

5 g Trypton
10 g Hefeextrakt
5 g Natriumchlorid
ad 1l Wasser

### Erste Phase: Anzucht

Für die Hauptkultur wurde 500 ml LB-Medium mit 30 µg Kanamycin pro ml Medium in einem 2 l- Schüttelkolben mit Schikanen mit 1% (v/v) Vorkultur angeimpft. Die Anzucht erfolgte bei 37°C bis zu einer OD₆₀₀ₙₘ von 0,6.

### Zweite Phase: Induktion und Expression

Nach Erreichen dieser OD erfolgte die Induktion mit 0,5 mM Isopropyl-β-D-thiogalactopyranosid (IPTG) und die anschließende Expression bei einer Temperatur von 22°C über Nacht (ca. 14h).

### Dritte Phase: Biotransformation

Die Zellsuspension wurde in ein steriles Zentrifugengefäß überführt und bei 8.000g und 4°C für 10 min zentrifugiert. Das überschüssige Medium wurde durch Dekantieren entfernt und das verbleibende Zellpellet zum Waschen in sterilem Umsetzungsmedium gelöst. Anschließend wurde erneut bei 8.000g und 4°C für 10 min zentrifugiert und das Umsetzungsmedium ebenfalls durch Dekantieren entfernt. Verbleibende Flüssigkeit wurde mit einer sterilen Pipette entfernt und das Zellpellet gewogen. Daraus ergab sich ca. 2 g Zellnassmasse pro 500 ml Kultur. Um eine Konzentration von 20 g Zellnassmasse pro I zu erhalten, wurde das Pellet in 100 ml sterilem Umsetzungsmedium resupendiert.

### Umsetzungsmedium

200 mM Kaliumphosphatpuffer
0,5 % (w/v) Glucose
pH 7,5

Die Biotransformation wurde in einem 300 ml-Schüttelkolben mit Schikane mit einem Volumen von 25 ml durchgeführt. Die Zugabe des Substrates Hexadecansäure erfolgte gelöst in Dimethylsulfoxid (DMSO). Dazu wurde 100 mM Hexadecansäure in DMSO gelöst und 1,25 ml dieser Lösung der Zellsuspension zugesetzt, sodass eine Endkonzentration von 5 mM im Biotransformationsansatz erreicht wurde. Vor Zugabe der Hexadecansäure wurde die Zellsupension im Schüttelkolben im Inkubator unter Schütteln auf 30°C vorgewärmt. Darüber hinaus wurde dem Biotransformationsansatz 1% (w/v) Triton X 100 zugesetzt.

Die Biotransformation fand bei 30°C unter starkem Schütteln statt. Nach ca. 3 h wurde eine Pentadecanalkonzentration von 4 mM erreicht. Das heißt der Umsatz betrug 80%.

### Wiederholung dritte Phase: Biotransformation mit zurückgewonnenem Biokatalysator

Dazu wurden die Zellen nach 3h bei Zimmertemperatur für 10 min bei 5000 g geerntet und der Überstand durch Dekantieren entfernt. Das verbleibende Zellpellet wurde zum Waschen in Umsetzungsmedium gelöst und anschließend erneut bei Zimmertemperatur für 10 min bei 5000 g zentrifugiert. Der Überstand wurde durch Dekantieren entfernt und das Zellpellet in 20 ml Umsetzungsmedium resuspendiert. Im Überstand konnte eine Produktkonzentration von 3 mM gemessen werden, das heißt ca. 75% des Produktes verbleibt nach dem Zentrifugieren im Überstand und kann daher vom Biokatalysator getrennt werden.

Für die erneute Biotransformation wurden die 20 ml Zellsuspension in einen frischen 300 ml Schüttelkolben mit Schikanen gegeben und 1 ml der oben beschriebenen Substratlösung (100 mM Hexadecansäure in DMSO) zugesetzt, so dass die Substratkonzentration im zweiten Durchlauf ebenfalls wieder ca. 5 mM betrug. Außerdem wurde auch hier wieder 1% (w/v) Triton X 100 zum Biotransformationsansatz gegeben. Bei diesem zweiten Durchlauf der Biotransformation konnte bereits nach 1 h eine Produktkonzentration von 5,5 mM erreicht werden. Zum Zeitpunkt t0=0 h dieser zweiten Biotransformation wurde eine Pentadecanalkonzentration von 1 mM gemessen (diese Reste sind aus der ersten Biotransformation trotz waschen im Pellet verblieben), das heißt es konnte im zweiten Durchlauf erneut ein Umsatz von 80-90% erzielt werden.

## Patentansprüche

1. Verfahren zum Herstellen eines Aldehyds mit 5 bis 31 Kohlenstoffatomen, umfassend die Schritte:
a) Bereitstellen von Mikroorganismenzellen enthaltend eine alpha-Dioxygenase,
b) Beaufschlagen der Mikroorganismenzellen mit einem Umsetzungsmedium enthaltend eine Fettsäure mit 6 bis 32 Kohlenstoffatomen,
c) Umsetzen der Fettsäure zum Aldehyd durch die alpha-Dioxygenase.

2. Verfahren nach Anspruch 1, **dadurch gekennzeichnet, dass** die alpha-Dioxygenase
a) eine Aminosäuresequenz-Ähnlichkeit ("Similarity") zu SEQ ID No. 1 von zumindest 80% gemessen mit dem Waterman-Smith-Algorithmus mit einer Lückeneröffnungs-Strafe ("Gap open penalty") von 10, einer Lückenfortsetzungsstrafe ("Gap extension penalty") von 0,5 und der Blosum62-Matrix, oder
b) eine Aminosäuresequenz gemäß SEQ ID No. 2 besitzt.

3. Verfahren nach einem der Ansprüche 1 oder 2, **dadurch gekennzeichnet, dass** die alpha-Dioxygenase eine Peroxidase-Aktivität von höchstens 0,4 nkat/mg Guaiacol/2-Hydroperoxyplamitinsäure besitzt.

4. Verfahren nach einem der Ansprüche 1, 2, oder 3, **dadurch gekennzeichnet,**
**dass** die in Schritt b eingesetzte Fettsäure ausgewählt ist aus
- linearen oder verzweigten gesättigten Fettsäuren,
- linearen oder verzweigten ungesättigten Fettsäuren,
- ungesättigte oder gesättigte Fettsäuren mit 1 bis 5 Substituenten, wobei die Substituenten jeweils unabhängig ausgewählt sind aus Hydroxy, C1-C10-Alkyl, C1-C10-Alkoxy, C6-C10-Aryl, Phenyl-C1-C5-Alkyl und Phenyl-C1-C5-Alkenyl, wobei das alpha-C-Atom der Fettsäure keinen solchen Substituenten trägt.

5. Verfahren nach Anspruch 4, **dadurch gekennzeichnet, dass** die in Schritt b eingesetzte Fettsäure und der zugeordnete in Schritt c erhaltene Aldehyd ausgewählt sind aus
| | |
|---|---|
| n-Hexadecansäure | n-Pentadecanal |
| n-Tetradecansäure | n-Tridecanal |
| n-Dodecansäure | n-Undecanal |
| n-Decansäure | n-Nonanal |
und Mischungen zweier oder mehrerer der genannten Fettsäuren zum Erhalten von Mischungen der entsprechenden Aldehyde.

6. Verfahren nach einem der Ansprüche 1, 2, 3, 4, oder 5, **dadurch gekennzeichnet, dass** die Mikroorganismenzellen ausgewählt sind aus Mikroorganismen
- der Klasse der Gamma-Proteobakterien,
- der Klasse der Bacilli, und
- der Klasse der Saccharomyceten.

7. Verfahren nach einem der vorherigen Ansprüche, **dadurch gekennzeichnet, dass** in Schritt b)
- ein pH-Wert des Umsetzungsmediums auf 6,5 bis 9, eingestellt wird, und/oder
- das Umsetzungsmedium einen Gehalt an Stickstoff-Verbindungen von höchstens 0,1 ppm besitzt, und/oder
- das Umsetzungsmedium einen Glucosegehalt von 0,25 - 1 % (w/v) besitzt, bezogen auf das gesamte Umsetzungsmedium.

8. Verfahren nach einem der vorherigen Ansprüche, **dadurch gekennzeichnet, dass** zum Umsetzen in Schritt c) eine Temperatur von 25-39°C eingehalten wird.

9. Verfahren zum Herstellen eines Aldehyds mit 5 bis 31 Kohlenstoffatomen, umfassend das Umsetzen einer Fettsäure mit 6 bis 32 Kohlenstoffatomen zum Aldehyd mit einem Mikroorganismus enthaltend eine alpha-Dioxygenase.

## Claims

1. Method for producing an aldehyde with 5 to 31 carbon atoms, comprising the steps:
a) Providing microorganism cells containing an alpha-dioxygenase,
b) Applying a conversion medium containing a fatty acid with 6 to 32 carbon atoms to the microorganism cells,
c) Converting the fatty acid to the aldehyde by means of the alpha-dioxygenase.

2. Method according to claim 1, **characterised in that** the alpha-dioxygenase has
a) an amino acid sequence similarity to SEQ ID No. 1 of at least 80% measured using the Waterman-Smith algorithm with a gap open penalty of 10, a gap extension penalty of 0.5 and the Blosum62 matrix, or
b) an amino acid sequence according to SEQ ID No. 2.

3. Method according to claim 1 or 2, **characterised in that** the alpha-dioxygenase has a peroxidise activity of at most 0.4 nkat/mg guaiacol/2-hydroperoxypalmitic acid.

4. Method according to any of the claims 1, 2 or 3, **characterised in that** the fatty acid used in step b is selected from
- linear or branched saturated fatty acids,
- linear or branched unsaturated fatty acids,
- unsaturated or saturated fatty acids with 1 to 5 substituents, wherein the substituents are in each case selected independently from hydroxy, C1-C10-alkyl, C1-C10-alkoxy, C6-C10-aryl, phenyl-C1-C5-alkyl and phenyl-C1-C5-alkenyl, wherein the alpha-C atom of the fatty acid does not carry any such substitutent.

5. Method according to claim 4, **characterised in that** the fatty acid used in step b and the associated aldehyde obtained in step c are selected from
| | |
|---|---|
| n-hexadecanoic acid | n-pentadecanal |
| n-tetradecanoic acid | n-tridecanal |
| n-dodecaonoic acid | n-undecanal |
| n-decanoic acid | n-nonanal |
and mixtures of two or more of the said fatty acids to obtain mixtures of the corresponding aldehydes.

6. Method according to any of the claims 1, 2, 3, 4 or 5, **characterised in that** the microorganism cells are selected from microorganisms
- of the class of gamma proteobacteria
- of the class of bacilli, and
- of the class of saccharomycetes.

7. Method according to any of the preceding claims, **characterised in that** in step b)
- a pH value of the conversion medium is adjusted to 6.5 to 9, and/or
- the conversion medium has a content of nitrogen compounds of at most 0.1 ppm, and/or
- the conversion medium has a glucose content of 0.25 - 1% (w/v) based on the total conversion medium.

8. Method according to any of the preceding claims, **characterised in that** for conversion in step c) a temperature of 25-39°C is maintained.

9. Method for producing an aldehyde with 5 to 31 carbon atoms comprising the conversion of a fatty acid with 6 to 32 carbon atoms to the aldehyde with a microorganism containing an alpha-dioxygenase.

## Revendications

1. Procédé pour fabriquer un aldéhyde avec 5 à 31 atomes de carbone comprenant les étapes :
a) Mise en place de cellules de microorganismes comprenant l'alpha-dioxygénase,
b) Application des cellules de microorganismes avec un milieu de réaction comprenant un acide gras avec 6 à 32 atomes de carbone,
c) Réaction de l'acide gras à l'aldéhyde par l'alpha-dioxygénase.

2. Procédé selon la revendication 1, **caractérisé par le fait que** l'alpha-dioxygénase a
a) une similarité de séquence d'aminoacide (« Similarity ») selon SEQ ID No. 1 d'au moins 80 % mesuré par l'algorithme de Waterman-Smith avec une pénalité de création de trous (« gap open penalty ») de 10, une pénalité d'extension de trous (« gap extension penalty ») de 0,5 et de la matrice de Blosum62, ou
b) une séquence d'aminoacide selon SQE ID No. 2.

3. Procédé selon la revendication 1 ou 2, **caractérisé par le fait que** l'alpha-dioxygénase a une activité de peroxydase d'au maximum 0,4 nkat/mg Guaiacol/acide 2-hydroxyperoxypalmitique.

4. Procédé selon la revendication 1, 2 ou 3, **caractérisé par le fait que** l'acide gras usé dans l'étape b) est choisi dans
- de acides gras saturés linéaires ou ramifiés,
- de acides gras non-saturés linéaires ou ramifiés,
- de acides gras non-saturés ou saturés avec 1 à 5 substituants, où les substituants sont choisis chacun indépendamment dans Hydroxy, C1-C10-Alkyl, C1-C10-Alkoxy, C6-C10-Aryl, Phenyl-C1-C5-Alkyl et Phenyl-C1-C5-Alkenyl, où l'alpha-C-atome de l'acide gras a pas desdits substituants.

5. Procédé selon la revendication 4, **caractérisé par le fait que** l'acide gras usé dans l'étape b) et l'aldéhyde correspondant et obtenu dans l'étape c) sont choisis dans
| | |
|---|---|
| d'acide n-hexadécanoïque | de n-Pentadecanal |
| d'acide n-tetradécanoïque | de n-Tridecanal |
| d'acide n-dodécanoïque | de n-Undecanal |
| d'acide n-décanoïque | de n-Nonanal |
et de mélanges de deux ou plusieurs desdits acides gras pour obtenir des mélanges des aldéhydes correspondants.

6. Procédé selon une des revendications 1, 2, 3, 4 ou 5, **caractérisé par le fait que** les cellules de microorganismes sont choisies par de microorganismes
- de la classe de gamma-protéobacteries,
- de la classe de bacilli, et
- de la classe de saccharomyces.

7. Procédé selon l'une quelconque des revendications précédentes **caractérisé par le fait que** dans l'étape b)
- un ph du milieu de réaction est ajusté à 6,5 à 9, et/ou
- le milieu de réaction a un contenu de composés azotés d'au maximum 0,1 ppm et/ou
- le milieu de réaction a un contenu de glucose de 0,25 - 1 % (w/v) par rapport au milieu de réaction total.

8. Procédé selon l'une quelconque des revendications précédentes, **caractérisé par le fait que** pendant la réaction dans l'étape c) la température est maintenue de 25 - 39 °C.

9. Procédé pour fabriquer un aldéhyde avec 5 à 31 atomes de carbone comprenant la réaction d'un acide gras avec 6 à 32 atomes de carbone à l'aldéhyde avec un microorganisme comprenant une alpha-diogénase.
